# EUROPÄISCHE PATENTSCHRIFT

(11) **EP 4 565 174 B1**
(45) Veröffentlichungstag und Bekanntmachung des Hinweises auf die Patenterteilung: **11.11.2026**
(21) Anmeldenummer: 23758521.1
(22) Anmeldetag: 01.08.2023
(51) Int. Cl.: A61C 19/06

(54) **APPLIKATIONSDÜSE ZUR AUSBRINGUNG EINES DENTALEN WIRKSTOFFS IM MUNDRAUM EINES PATIENTEN SOWIE WIRKSTOFFAPPLIKATOR MIT EINER DERARTIGEN APPLIKATIONSDÜSE**
APPLICATION NOZZLE FOR DISCHARGING A DENTAL ACTIVE SUBSTANCE IN THE ORAL CAVITY OF A PATIENT, AND ACTIVE SUBSTANCE APPLICATOR HAVING SUCH AN APPLICATION NOZZLE
BUSE D'APPLICATION POUR FAIRE SORTIR UN PRINCIPE ACTIF DENTAIRE DANS LA CAVITÉ BUCCALE D'UN PATIENT ET APPLICATEUR DE PRINCIPE ACTIF DOTÉ D'UNE TELLE BUSE D'APPLICATION

(30) Priorität: 03.08.2022 DE 102022119523; 07.10.2022 DE 102022210641
(43) Veröffentlichungstag der Anmeldung: 11.06.2025
(73) Patentinhaber: Institut Straumann AG, 4002 Basel (CH)
(72) Erfinder: ZIPPRICH, Holger, 64342 Malchen (DE)
(74) Vertreter: Tergau & Walkenhorst Intellectual Property GmbH
(86) Internationale Anmeldenummer: PCT/EP2023/071323
(87) Internationale Veröffentlichungsnummer: WO 2024/028344

(56) Entgegenhaltungen:
- US-A1- 2008 145 814
- US-A1- 2008 145 814
- US-A1- 2019 015 180
- US-A1- 2019 015 180
- US-B2- 7 530 808
- US-B2- 7 530 808

## Beschreibung

Die Erfindung betrifft eine Applikationsdüse zur Ausbringung eines dentalen Wirkstoffs im Mundraum eines Patienten. Sie bezieht sich weiter auf einen Wirkstoffapplikator mit einer derartigen Applikationsdüse.

In einer Vielzahl von Situationen bei der dentalen Behandlung oder der Vor- oder Nachsorge besteht das Bedürfnis, zielgenau und präzise einen dentalen Wirkstoff, beispielsweise ein Spülmittel, ein Desinfektionsmittel, ein Medikament oder dergleichen, im Mundraum des Patienten zu platzieren. Dabei kann es sich um Maßnahmen der Zahnpflege oder auch um therapeutische Maßnahmen, beispielsweise im Rahmen einer Vor- oder Nachsorge, zum Beispiel im Rahmen einer Parodontosebehandlung, handeln. Es besteht generell der Wunsch, ein einfach gehaltenes Applikationsinstrument bereitzustellen, mit dem eine solche zielgenaue Verabreichung im Patientenmund auf einfache Weise ermöglicht ist. Im Hinblick auf den Umstand, dass dabei neben therapeutischen auch prophylaktische Maßnahmen oder Pflegemaßnahmen ermöglicht werden sollten, sollte für ein solches Applikationsinstrument das zur eigentlichen Verabreichung im Patientenmund vorgesehene Bauteil, also die so genannte Applikationsdüse, insbesondere auch einfach und kostengünstig und damit auch für große Stückzahlen geeignet herstellbar sein. Des Weiteren sollte ein solches Applikationssystem auch für medizinisch nicht geschulte Anwender, insbesondere im Rahmen der Selbstbehandlung, einfach und zuverlässig nutzbar sein, von der Einfachheit her beispielsweise wie eine Zahnbürste. Applikationsdüsen zur Ausbringung eines dentalen Wirkstoffs im Mundraum eines Patienten sind beispielsweise aus der US 2019/015180 A1, der US 2008/145814 A1 oder der US 7,530,808 bekannt.

Weiterhin besteht insbesondere im Zahnpflege- oder Vor- oder Nachsorgebereich der Wunsch, in besonders betroffenen Bereichen wie beispielsweise den Zahntaschen eine besonders intensive Spülwirkung erzeugen zu können, insbesondere um Speisereste oder Verunreinigungen, wie z.B. Biofilme oder deren Ausscheidungen mit hoher Effizienz austragen und damit entsprechenden Bakterien- oder Keimbefall besonders gering zu halten.

Der Erfindung liegt nunmehr die Aufgabe zu Grunde, eine Applikationsdüse zur Ausbringung eines dentalen Wirkstoffs im Mundraum eines Patienten anzugeben, mit der diesen Aspekten Rechnung getragen wird. Des Weiteren soll ein insbesondere auch für die Nutzung durch Privatpersonen geeigneter Wirkstoffapplikator angegeben werden.

Bezüglich der Applikationsdüse wird diese Aufgabe erfindungsgemäß gelöst mit einem Düsenkörper, der als Laminat aus einer Mehrzahl von Folienstücken aufgebauter Schichtenkörper ausgebildet ist.. Um im Hinblick auf die angestrebte Verwendung der Applikationsdüsen im Zahnpflege- und Prophylaxe-Bereich die Herstellung einer enorm großen Stückzahl derartiger Applikationsdüsen und somit eine Massenproduktion zu ermöglichen, ist zudem eine hierfür besonders geeignete Bauweise gewählt, die auch bei der Verwendung vergleichsweise kostengünstiger Materialien die Bereitstellung eines funktionell zuverlässigen Applikationskonzepts erlaubt. Um dem Rechnung zu tragen, ist als Basismaterial für die Herstellung des Grundkörpers der Düse ein Folienmaterial vorgesehen, das durch Laminieren oder Verschweißen einer Mehrzahl von Folienschichten übereinander zu einem geeigneten Verbundkörper aufgebaut werden kann und auch einen Vorratsbehälter und/oder die Medienkanäle beinhalten kann. Der Grundkörper oder Düsenkörper, in dem die auslassseitig in jeweils einer Applikationsöffnung mündenden Medienkanäle angeordnet sind, ist somit von einem als Laminat oder als Verschweißung aus einer Mehrzahl von Folienstücken aufgebauten Schichtenkörper gebildet.

Die zum Transport des zu verabreichenden Wirkstoffs an die vorgesehene Abgabestelle in diesem Verbundkörper vorgesehen Medien-Transportkanäle, die ausgangsseitig in der Mischkammer des Verbundkörpers münden, können dabei durch in die jeweilige Folie eingebrachte Aussparungen bereitgestellt werden. Gerade der Rückgriff auf eine auf Folien basierende Technik mit anschließendem Laminieren oder Verschweißen ermöglicht dabei eine enorme Flexibilität bei der Auslegung und Einbringung solcher Medienkanäle, da die für diese erforderlichen Frei- oder Leerräume innerhalb des Verbundkörpers durch geeignete Formgebung in der jeweiligen Folie in vielfältiger Weise und verschiedensten Geometrien erzeugt werden können. Die Formgebung erfolgt dabei vorteilhafterweise durch das besonders bevorzugt vorgesehene Ausstanzen, Lasern oder Prägen; alternativ kann aber auch die Bearbeitung durch Fräsen oder Ätzen (im Falle nasschemisch auflösbaren Folienmaterials) vorgesehen sein. Die Folie, in die die Aussparungen eingebracht werden, kann dabei vorzugsweise auf einer Trägerfolie angebracht sein, die ihrerseits als Teil des Folienstapels vorgesehen sein oder später, nach Einbringung der Aussparungen, abgezogen werden kann. Beim Prägen, oder insbesondere beim Heißprägen, kann beispielsweise auch eine dualextrudierte Folie verwendet werden, in die mit einem heißen Stempel oder einer Rolle die Medienkanäle eingebracht werden.

In einer als eigenständig erfinderisch angesehenen Ausgestaltung münden die Medienkanäle auslassseitig in jeweils einer Applikationsöffnung. In einer weiteren, als eigenständig erfinderisch angesehenen Ausgestaltung der Applikationsdüse wird die genannte Aufgabe erfindungsgemäß gelöst mit einem Düsenkörper, in den mindestens zwei unabhängig voneinander vom Anschlussbereich hin zu einer Mischstelle verlaufende und in dieser zusammenlaufende Medienkanäle integriert sind, wobei die Mischstelle auslassseitig über einen weiteren Medienkanal mit einer am freien Behandlungsende vorgesehenen Applikationsöffnung verbunden ist.

Insbesondere sind dabei die Mischstelle und/oder der weitere, in der Applikationsöffnung mündende Medienkanal ebenfalls in den Düsenkörper integriert. Vorteilhafte Ausgestaltungen der Erfindung sind Gegenstand der Unteransprüche. Weitere und/oder alternative vorteilhafte Ausgestaltungen der Erfindung ebenso wie weitere als eigenständige Erfindungen angesehene Ausgestaltungen ergeben sich auch aus der Figurenbeschreibung.

Gemäß einem Aspekt der Erfindung sollen somit über die Applikationsdüse zwei (oder mehr) medienseitig voneinander getrennt gehaltene Zuführkanäle bereitgestellt werden, über die unterschiedliche Wirkstoffe vermischungsfrei und unabhängig voneinander in einen gemeinsamen Raumbereich in der Nähe des freien Behandlungsendes ausgebracht werden können. Die Erfindung geht dabei von der Überlegung aus, dass durch eine solche Bauweise nicht nur Wirkstoffe an sich, sondern auch Reaktionen zwischen zwei Wirkstoffen gezielt in einer lokalisierten Position im Mundraum, beispielsweise in einer Zahntasche, zum Einsatz gebracht werden können. Damit können nicht nur die Wirkstoffe selbst, sondern auch die Reaktionsprozesse zwischen ihnen und dabei eventuell freigesetzte Reaktionsprodukte zum Zweck einer Behandlung und für definierte Ziele einsetzbar gemacht werden.

Beispielsweise können dabei gemäß einem als eigenständig erfinderisch angesehenen Aspekt der Erfindung Wirkstoffe oder Komponenten direkt am Applikationsort zusammengebracht werden, die unter Bläschenbildung miteinander reagieren, wobei die entstehenden Gasblasen zum Austrag und zur Mitnahme von Verunreinigungen, Speiseresten und dergleichen genutzt werden können. Ein solches Konzept eignet sich somit beispielsweise zur Nutzung in einem gezielten Pflege- und Reinigungsprogramm für die Zähne, bei dem auch die Zahntaschen mitbehandelt und mit gereinigt werden. Beispielsweise und gemäß einem als eigenständig erfinderisch angesehenen Aspekt der Erfindung könnten als Wirkstoffe oder Komponenten einerseits ein Hydrogencarbonat, vorzugsweise ein Natrium- oder Kaliumhydrogencarbonat, und andererseits eine Säure, vorzugsweise einer Carbonsäure (z. B. Zitronensäure oder Milchsäure) vorgesehen sein. Diese werden dann gemeinsam ausgebracht, kommen am Applikationsort in Kontakt miteinander und reagieren unter Bläschenbildung (CO₂-Bildung). Dieser Effekt könnte beispielsweise zu Reinigungs- oder Spülzwecken besonders günstig eingesetzt werden.

Um die gemäß einem Aspekt der Erfindung vorgesehene Nutzung von Reaktionseffekten zwischen zwei oder mehr Wirkstoffen oder Komponenten besonders zuverlässig und zudem zielgenau in einem gewählten Applikationsbereich, beispielsweise einer Zahntasche, zu ermöglichen, sind vorteilhafterweise die den beiden Medienkanälen jeweils zugeordneten Austritts- oder Applikationsöffnungen möglichst nahe beieinander positioniert, so dass die beabsichtigte Vermischung der Komponenten zuverlässig und gezielt im vom Benutzer durch die Positionierung der Behandlungsspitze ausgewählten Raumbereich stattfindet. Somit sind in einer vorteilhaften Ausgestaltung die den beiden Medienkanälen zugeordneten Applikationsöffnungen mindestens 0,01 mm und höchstens 10 mm, vorzugsweise mindestens 0,1 mm und/oder höchstens 2 mm, in ganz besonders vorteilhafter Ausgestaltung höchstens 0,1 mm bis 0,6 mm, voneinander beabstandet.

Gemäß einem alternativen, als eigenständig erfinderisch angesehenen Aspekt der Erfindung sollen hingegen über die Applikationsdüse zwei (oder mehr) medienseitig voneinander getrennt gehaltene Zuführkanäle bereitgestellt werden, über die unterschiedliche Wirkstoffe vermischungsfrei und getrennt voneinander in einen gemeinsamen Raumbereich, nämlich eine in den Düsenkörper integrierte Mischstelle, ausgebracht werden können. Damit kann die Vermischung der Wirkstoffe kontrolliert und unter einstellbaren Randbedingungen vorgenommen werden. Damit können gezielt Effekte genutzt werden, die durch die Vermischung ausgelöst werden, beispielsweise Reaktionen zwischen den Wirkstoffen. Insbesondere kann dabei vorgesehen sein, gezielt eine Reaktion auszulösen, die ausgehend von ursprünglich flüssigen Wirkstoffen mit Gasbildung und entsprechend einer erheblichen Druckerhöhung einhergeht. Beispielsweise und gemäß einem Aspekt der Erfindung könnten als Wirkstoff einerseits ein Hydrogencarbonat, bevorzugt Kalium-, Natrium- oder Calciumhydrogencarbonat, besonders bevorzugt Natriumhydrogencarbonat, und andererseits eine Säure / Carbonsäure (z. B. Äpfelsäure, Zitronensäure, Milchsäure etc.) vorgesehen sein, vorzugsweise jeweils in wässriger Lösung und in für die angestrebte Handhabung geeigneter Konzentration. Diese werden dann gemeinsam der Mischstelle zugeführt, kommen dort in Kontakt miteinander und reagieren unter Bläschenbildung (CO₂-Bildung). Damit kann in der Mischstelle in Folge dieser Reaktion unter Bläschenbildung ein sehr hoher Druck ohne aktive Einflussnahme von außen erzeugt werden, so dass das Medium aus der Mischstelle mit vergleichsweise hohem Druck und im Ergebnis als gerichteter Strahl aus der Applikationsöffnung des Düsenkörpers austritt. Dieser Effekt könnte beispielsweise zu Reinigungs- oder Spülzwecken besonders günstig eingesetzt werden, wobei gerade die Erzeugung eines gerichteten Strahls unter hohem Druck besonders gut nutzbar ist.

Die Erfindung geht im Umfang dieses Aspekts somit von der Überlegung aus, dass zur Ermöglichung der erwünschten besonders hohen Reinigungs- oder Spülwirkung ein gerichteter, unter Druck stehender Medienstrahl bereitgestellt werden sollte. Dies ist auf besonders einfache "selbsttätige" Bauweise ohne die Notwendigkeit äußerer Eingriffe durch die genannte Bauweise möglich, die eben die Auslösung von Reaktionen zwischen zwei Wirkstoffen gezielt in einer lokalisierten Position im Düsenkörper ermöglicht. Damit können nicht nur die Wirkstoffe selbst, sondern auch die Reaktionsprozesse zwischen ihnen und dabei eventuell freigesetzte Reaktionsprodukte zum Zweck einer Behandlung und für definierte Ziele einsetzbar gemacht werden. Insbesondere durch geeignete Formgebung der Kammergeometrie und/oder der Geometrien der Medienkanäle sowie ggf. die Bereitstellung weiterer Komponenten wie beispielsweise Ventilklappen ist dabei gemäß einem Aspekt der Erfindung eine gezielte Vorgabe der Druckverhältnisse oder anderer Parameter im entstehenden Medienstrahl möglich.

Ein solches Konzept eignet sich somit beispielsweise zur Nutzung in einem gezielten Pflege- und Reinigungsprogramm für die Zähne, bei dem auch die Zahntaschen mitbehandelt und mit gereinigt werden.

Vorteilhafterweise ist die Mischstelle dabei als Mischkammer ausgestaltet, zu der hin die Medienkanäle verlaufende und in die sie münden. Die Mischkammer kann dabei insbesondere mittels geeigneter Geometrie- oder Volumenwahl gezielt an die angestrebte Reaktionsdynamik angepasst werden; vorteilhafterweise weist sie ein Volumen von höchstens 1000 mm³, vorzugsweise von höchstens 500 mm³, besonders bevorzugt von höchstens 100 mm³, auf.

Die Medienkanäle und/oder die Mischkammer können dabei insbesondere durch den vorgesehenen Rückgriff auf die Laminier-, Schweiß- oder Prägetechnik hergestellt werden, indem beispielsweise eine zentrale Folie mit entsprechenden Aussparungen hergestellt und anschließend ober- und unterseitig jeweils mit einer durchgehenden Folie zu einem Verbundkörper in der Art eines Foliensandwich zusammenlaminiert oder -geschweißt wird oder zwei symmetrische Prägungen aufeinander laminiert oder geschweißt werden.

Vorteilhafterweise ist als Basismaterial für die Folien oder Folienstücke Polyamid vorgesehen; alternativ kann aber auch ein anderes geeignetes Folienmaterial wie PVC, PP oder PE oder auch eine Kombination unterschiedlicher Folienmaterialien als günstig angesehen werden. Die Materialwahl erfolgt dabei gemäß einem Aspekt der Erfindung insbesondere im Hinblick darauf, dass die Applikationsdüse zwar auch für den Gebrauch durch geschultes medizinisches Personal, beispielsweise im Zusammenhang mit einer Behandlung durch den Zahnarzt, aber andererseits auch für den Gebrauch durch in der Regel nicht sonderlich geschulte Privatpersonen, insbesondere den Patienten selbst im Rahmen der Zahnpflege, geeignet sein sollte. Besonders bevorzugt ist das Folienmaterial dabei hinsichtlich seiner Materialeigenschaften wie insbesondere seiner Steifigkeit oder Festigkeit derart gewählt, dass die Steifigkeit auch des aus den Folienstücken gebildeten Laminat- oder Verbundkörpers nicht zu groß ist und somit Verletzungen im Mundraum weitgehend ausgeschlossen sind.

In ganz besonders vorteilhafter, als eigenständig erfinderisch angesehener Ausgestaltung ist der Düsenkörper dabei aus mindestens drei Folienschichten aufgebaut, die sich in ihren Materialeigenschaften unterscheiden und funktional an unterschiedliche Vorgaben geeignet angepasst sind. Insbesondere kann gemäß einem Aspekt der Erfindung eine zentrale, zwischen zwei benachbarten Folienschichten angeordnete Folienschicht aus einem härteren Folienmaterial, also insbesondere mit unterschiedlicher Shore-Härte oder E-Modul, ausgebildet sein als die beiden benachbarten Folienschichten. Damit kann die zentrale Folienschicht formgebend in der Art einer Stützstruktur die Kontur oder Raumform des Düsenkörpers festlegen, wohingegen die vergleichsweise weicheren äußeren Folienschichten nachgiebig und verformbar ausgestaltet sein können und damit das Verletzungsrisiko bei einem Kontakt beispielsweise der Mundschleimhaut mit dem Düsenkörper deutlich verringern. Sind Düsenkörper, Schlauch und Vorratsbehälter in einem, ist in einer besonders vorteilhaften Ausführung die mittlere und steifere Folie lediglich in dem Düsenkörper angebracht.

Gemäß einem Aspekt der Erfindung ist der Düsenkörper aus mindestens drei Folienschichten aufgebaut, in denen jeweils ein Lichtleiter oder eine Lichtleiterfolie integriert ist. Dieser Aufbau kann gemäß einem als eigenständig erfinderisch angesehenen Ansatz zur Zahn- bzw. Weichgewebs-Seitenerkennung genutzt werden. Dabei ist insbesondere vorgesehen, durch einen zentral angeordneten Lichtleiter weißes Licht in das Folienpaket und somit in den Applikationsbereich, beispielsweise in der Zahntasche, einzuleiten. Dieses wird dort reflektiert, und das reflektierte Licht kann dann in außenseitig angeordneten Lichtleitern wieder zurückgeleitet werden. Falls dabei die Reflektion an Zahn- oder Implantatmaterial erfolgte, ist im reflektierten Licht der Rotanteil geringer als wenn die Reflektion am Weichgewebe erfolgt ist. Durch Vergleich der Rotanteile im reflektierten Licht kann somit erkannt werden, welche Seitenfläche des Düsenkörpers dem Zahn bzw. Implantat zugewandt ist und welche dem Weichgewebe. Unter Nutzung dieser Erkenntnis kann dann die Bespeisung der beidseitig von der Zentralfolie angeordneten Medienkanäle besonders bedarfsgerecht erfolgen. Eine Unterscheidung zwischen Zahn und Implantat kann darüber hinaus auch noch erfolgen, indem der Helligkeitsunterschied ermittelt wird: das vergleichsweise hellere reflektierte Licht wird dabei dem Zahn zugeordnet; das vergleichsweise dunklere dem Implantat.

Gemäß einem weiteren, als eigenständig erfinderisch angesehenen Aspekt der Erfindung sind die äußeren Schichtfolien des Folienpakets vorteilhafterweise eingefärbt, wobei eine erste Außenfolie weiß und die zweite Außenfolie rot eingefärbt ist. Diese Ausgestaltung dient zur Arbeitserleichterung für den Benutzer bezüglich der korrekten Ausrichtung der Applikationsdüse: die rot eingefärbte Außenfolie soll dabei bei korrektem Gebrauch zum Weichgewebe oder Zahnfleisch hin zeigen und die weiße zum Zahn. Bei einer solchen Ausgestaltung ist in vorteilhafter Weiterbildung nur die weiße, also zum Zahn hin auszurichtende, Seite mit Applikationsöffnungen versehen. Damit kann sichergestellt werden, dass beispielsweise ein Desinfektions- oder Behandlungsmittel nur auf den Zahn, nicht aber ins Weichgewebe ausgebracht wird.

Gemäß einem Aspekt der Erfindung werden die Medienkanäle durch die jeweils eingebrachte Aussparung in einer mittleren Folie gebildet, die seitlich durch die jeweiligen Seitenkanten begrenzt ist. Ober- und unterseitig wird der jeweilige Medienkanal dann durch die entsprechend auflaminierte oder aufgeschweißte, durchgehende Basis- bzw. Deckfolie begrenzt. Um dabei bedarfsweise auch vergleichsweise große, für den Durchtritt auch größerer Wirkstoffmengen geeignete freie Querschnitte der jeweiligen Medienkanäle mit entsprechend breit ausgelegten Aussparungen in der Mittel- oder Zentralfolie sicher und zuverlässig bereitstellen zu können, ist in vorteilhafter und als eigenständig erfinderisch angesehener Ausgestaltung eine Anzahl der Medienkanäle mit integrierten Abstandshaltern versehen.

In ganz besonders vorteilhafter und ebenfalls als eigenständig erfinderisch angesehener Ausgestaltung ist die Applikationsdüse auch hinsichtlich ihrer geometrischen Ausgestaltung und Dimensionierung an die vorgesehene Verwendung zur zielgenauen Einbringung von Wirkstoffen in den Mundbereich eines Patienten oder auch zum Spülen im Mundbereich ausgelegt. Dabei ist vorteilhafterweise insbesondere berücksichtigt, dass eine Ausbringung auch in die Zahnzwischenräume oder die Zahntaschen des Patienten vorgesehen sein könnte, insbesondere auch bei Zahnpflegemaßnahmen. Eine hierfür besonders geeignet ausgestaltete Applikationsdüse weist gemäß einem Aspekt der Erfindung einen als Laminat aus den Folienstücken aufgebauter Düsenkörper mit einer Gesamtdicke von 0,3 - 2 mm, vorzugsweise von 0,5 - 1,5 mm, besonders bevorzugt von 0,7 - 1,2 mm, auf. Hierzu korrespondierend weisen die das Laminat bildenden Folienstücke vorteilhafterweise jeweils eine Foliendicke von 50 - 500 µm, vorzugsweise von 80 - 350 µm, besonders bevorzugt von 100 - 250 µm, auf.

Alternativ oder zusätzlich ist gemäß einem Aspekt der Erfindung die Applikationsdüse auch hinsichtlich ihrer Formgebung besonders günstig an die genannten Anwendungsfälle, also die zielgenaue Ausbringung eines Wirkstoffs, zum Spülen im Mundraum und/oder allgemein zur Zahnpflege, geeignet angepasst. Dazu kann sich ihr Düsenkörper in einer Längsrichtung von einer Anschlussseite aus zu einem freien Behandlungsende hin flächig erstrecken, wobei er sich in seinem Querschnitt in Richtung auf das Behandlungsende hin verjüngt. Damit nimmt er näherungsweise die Kontur einer Behandlungsspitze an, so dass die Handhabung gerade in der vergleichsweise beengten Situation im Mundraum besonders erleichtert ist. Gemäß einem Aspekt der Erfindung kann somit die Ausführung der Applikationsdüse als im wesentlichen flächiges Bauteil vorgesehen sein. Dies soll nicht bedeuten, dass die Applikationsdüse zweidimensional ausgeführt sein könnte oder sollte; vielmehr ist hierunter zu verstehen, dass die Applikationsdüse bzw. der diesen bildende Grundkörper ein im Wesentlichen entlang einer basalen Ebene oder Grundebene ausgedehnter Körper sein sollte, der in der dritten Raumrichtung dennoch eine gewisse Dicke aufweist. Im Querschnitt gesehen bedeutet dies aber auch, dass die laterale Ausdehnung des Grundkörpers in der Basalebene deutlich größer ist als die Dicke in der Richtung senkrecht dazu. Für die Anwendung bedeutet dies, dass das freie oder Behandlungsende dieses Grundkörpers vergleichsweise einfach in die genannten Zahntaschen einbringbar ist, indem die Basalebene des Grundkörpers beispielsweise im Wesentlichen parallel zur Außenoberfläche des Zahns ausgerichtet wird.

Insbesondere kann der Grundkörper dabei, besonders im Bereich des Behandlungsendes, eine schwert- oder blattartige Raumform aufweisen, mit der sowohl die Zahntaschen als auch Zwischenräume zwischen den Zähnen gut erreichbar sind. Durch eine seitlich abstrahlende Applikationsöffnung ist dabei zudem erreichbar, dass auch Hinterschneidungen an den Zahnoberflächen, beispielsweise durch konkave Segment im Bereich der Zahnzwischenräume, gut und zuverlässig mit Medium beaufschlagt werden können.

Vorzugsweise ist die Applikationsöffnung bzw. sind die Applikationsöffnungen und somit die durch diese gebildete "Doppel-Düse" dabei in einem im Bereich des Behandlungsendes angeordneten Ausströmbereich des Düsenkörpers angeordnet. Hinsichtlich der Abströmrichtung aus der jeweiligen Applikationsöffnung kann diese jeweils für eine im Wesentlichen parallel zur Längsrichtung des Düsenkörpers ausgerichtete Abströmung des Mediums aus der Applikationsöffnung, oder aber auch für eine schräge oder seitliche Abströmung ausgelegt sein. Der Mündungsbereich des jeweiligen Medienkanals kann dabei geradlinig ausgerichtet oder auch gekrümmt oder abgewinkelt ausgestaltet sein. Weiterhin weist vorzugsweise der Düsenkörper in Draufsicht eine in der Art eines Dreiecks geformte Kontur auf, welche zur optimierten Anwendung auch als eine Art gekrümmtes Dreieck ausgeführt sein kann.

Neben den Ausstanzungen, die die Medienkanäle bilden, besteht auch die Möglichkeit, eine Prägung einzubringen, welche als Medienkanal fungiert.

Gemäß einem weiteren, als eigenständig erfinderisch angesehenen Aspekt der Erfindung ist die Applikationsdüse gezielt für die Nutzung im Rahmen der Zahnpflege ausgelegt, wobei eine für den Benutzer einfach gehaltene, zuverlässige Spülung auch schwierig zugänglicher Zonen wie beispielsweise der Zahntaschen besonders bedeutsam sein kann. Um dies besonders zu begünstigen, ist vorteilhaftweise zusätzlich zu einem auslassseitig in eine Applikationsöffnung mündenden, als Applikations-Medienkanal vorgesehenen Medienkanal mindestens ein weiterer Medienkanal vorgesehen, der als Absaugkanal ausgebildet ist. Der Applikations-Medienkanal kann dann zur zielgenauen Zuführung des Spül-Mediums beispielsweise in die Zahntasche genutzt werden, wobei die dadurch infolge der Spülung ausgetragenen Partikel, Speisereste oder Verschmutzungen mittels des oder der Absaugkanäle gezielt abgeführt oder entfernt werden können. Dies kann insbesondere während einer zahnärztlichen Behandlung sehr nützlich sein, wobei der Absaugkanal gemäß einem Aspekt er Erfindung an einen Speichelzieher, den Absauger am Patientenstuhl, an eine chirurgische Absaugeinheit oder eine andere externe Absaugung angeschlossen sein kann.

Gemäß einem weiteren, als eigenständig erfinderisch angesehenen Aspekt weist die Applikationsdüse zusätzlich zu den Medienkanälen einen ebenfalls in den Düsenkörper integrierten und mit der Mischstelle verbunden Druckkanal auf. Dieser kann insbesondere mit einer externen Druckquelle, beispielsweise einer Pumpe, verbunden oder verbindbar sein, so dass die Mischstelle zur Unterstützung des Druckaufbaus bei der Ausbringung des Mediums zusätzlich gezielt mit Druck beaufschlagt werden kann. Als Druckmedium kann dabei Luft, Wasser oder ein Gemisch daraus, ggf. auch versetzt mit Pulver oder Partikeln, vorgesehen sein. Die Druckbeaufschlagung erfolgt dabei vorteilhafterweise gepulst oder stoßweise, so dass bedarfsweise eine besonders intensive Ausbringung des Mediums erzeugt werden kann.

In vorteilhafter Weiterbildung ist die Applikationsdüse nach der vorstehend beschriebenen Art als Wegwerf- oder Einmal-Produkt ausgelegt und somit zum lediglich einmaligen Gebrauch vorgesehen.

In als eigenständig erfinderisch angesehener Weise kann der Düsenkörper, in ansonsten zur vorstehend beschriebenen Auslegung weitgehend identischer Ausgestaltung, anstelle einer Applikationsdüse auch als Zwischenstück ausgestaltet sein, bei dem die Einbringung der Medien in den Mundraum des Patienten nicht über am Behandlungsende des Düsenkörpers angeordnete Applikationsöffnungen, sondern über ein eigenständiges Ausbringungsteil, beispielsweise ein Schlauchpaket, einen Behandlungsschnorchel oder dergleichen, erfolgt. Bei einer solchen Ausgestaltung kann das eigentliche Ausbringungsteil geeignet an den Auslassbereich des Düsenkörpers angeschlossen werden, wobei ein in der Mischstelle oder insbesondere in der Mischkammer erzeugtes, unter Druck stehendes Behandlungsmedium über eine entsprechende, im Ausbringungsteil vorgesehene Druckleitung an den vorgesehenen Ausbringungsort im Mundraum des Patienten geleitet werden kann. Entsprechend können dabei für weitere im Düsenkörper geführte Medien, wie beispielsweise Einzelmedien, die erst an der Ausbringungsstelle im Mundraum des Patienten in Kontakt zueinander gebracht werden sollen, geeignete weitere Medienkanäle im Ausbringungsteil vorgesehen sein.

Hinsichtlich des Wirkstoffapplikators wird die genannte Aufgabe gelöst mit einer Applikationsdüse einer der vorstehend beschriebenen Bauarten, wobei jeder der beiden in eine Auslassöffnung bzw. in die Mischkammer mündenden Medienkanäle medienseitig jeweils mit einer separat zugeordneten Wirkstoffkammer verbunden ist.

Durch diese Bauweise wird die mit der Erfindung angestrebte Nutzung ermöglicht, dass die in den Wirkstoffkammern vorgehaltenen Wirkstoffe separat und unabhängig voneinander gemäß einer Ausführungsform der Erfindung über die Applikationsöffnungen an der vorgesehenen Position ausgebracht und erst dort zur Vermischung gebracht oder, gemäß einer alternativen Ausführungsform der Erfindung, zur Vermischung in die Mischkammer zugeführt werden können. Die Ausgestaltung kann dabei als Kartusche mit Düse zum händischen Auspressen, als Handgerät analog einer elektrischen Zahnbürste oder auch als Standgerät mit Schlauch zum Hand- / Mundstück, ggf. auch mit Licht, vorgesehen sein.

Der Wirkstoffapplikator kann dabei die Applikationsdüse als separates, eigenständiges Bauteil umfassen, das über geeignete Anschlussstellen mit den Wirkstoffkammern verbunden ist. Diese könnten dabei ortsfest installiert oder in der Art eines eigenständigen Bauteils bereits herstellungsseitig mit der Applikationsdüse verbunden sein, so dass in der Art eines vorkonfektionierten Systems die Applikationsdüse gemeinsam mit den bereits angeschlossenen Wirkstoffkammern an den Nutzer ausgeliefert wird. Auf diese Weise ist beispielsweise die portionierte, vordosierte Abgabe des Wirkstoffs an den Nutzer ermöglicht, wobei insbesondere auch alle Vorgaben aus dem Hygienebereich, beispielsweise notwendige Desinfektions- oder Sterilisationsmaßnahmen, unter Rückgriff auf die hierfür üblichen Konzepte wie beispielsweise entsprechende Umverpackungen problemlos eingehalten werden können. Die Applikationsdüse kann dabei als ersetzbares Wegwerfteil ausgeführt sein, wobei für einen Einsatz zunächst eine neue, unverbrauchte Düse an die eigentliche Apparatur angeschlossen wird.

Gemäß einem Aspekt der Erfindung kann der Wirkstoffapplikator aber auch in einer integrierten Bauweise in dem Sinne einstückig ausgeführt sein, dass die Wirkstoffkammern gemeinsam mit dem Düsenkörper in einen Grundkörper und gegebenenfalls gemeinsam mit dem Verbindungsschlauch integriert sind. Die "Applikationsdüse" bildet in einer derartigen Bauweise den dem Behandlungsende zugewandten Endabschnitt dieses gemeinsamen Grundkörpers, stellt somit also kein körperlich eigenständiges Bauteil dar. Als besonders vorteilhaft wird in dieser Bauweise die Ausgestaltung des gemeinsamen Grundkörpers als Mehrschicht-Folienkörper oder Laminat der oben beschriebenen Bauweise angesehen. Durch geeignete Formgebung der Folienschichten oder Folienstücke können dabei sowohl die Medienkanäle als auch die Wirkstoffkammern und gegebenenfalls auch die Verbindungselemente in den Körper integriert sein, wobei durch Nutzung der Folieneigenschaften und die dadurch gegebene Flexibilität die geeignete räumliche Ausgestaltung bedarfsgerecht gewählt werden kann. Insbesondere kann der Applikator dabei in der Art einer Ampulle ausgestaltet sein, wobei unter Nutzung der flexiblen Eigenschaften der jeweiligen Folien und der von diesen gebildeten Wirkstoffkammern diese zum Ausdrücken für den Patienten zu Hause geeignet sind. Der Applikator kann analog einer Zahnbürste mit Ampulle für den Patienten für zu Hause oder den Zahnarzt bereitgestellt werden.

Gemäß einem weiteren, ebenfalls als eigenständig erfinderisch angesehenen Aspekt der Erfindung kann die Applikationsdüse auch zur Verabreichung vordosierter oder portionierter Wirkstoffmengen ausgestaltet sein. Dazu ist gemäß einem Aspekt der Erfindung die jeweilige Wirkstoffkammer vorteilhafterweise mit einem an die zu verabreichende Patientendosis angepassten Innenvolumen ausgeführt. In dieser kann dann eine vordefinierte Dosis an Wirkstoff, z. B. Chlorhexidin zur Desinfektion oder eine Spüllösung, enthalten sein. Der Patient kann diese dann selbst zu Hause applizieren, wobei einerseits durch die Bauart der Applikationsdüse bedingt eine korrekte Positionierung im Mundraum ermöglicht wird und andererseits durch die portionierte Vorhaltung im Düsenkörper die korrekte Dosierung des Wirkstoffs sichergestellt werden kann. Die Ausbringung des in der Wirkstoffkammer vorgehaltenen Wirkstoffs bzw. dessen Einbringung in die Wirkstoffkammer kann dann, ggf. unter Nutzung der materialbedingt gegebenen Verformbarkeit des Folien-Verbundkörpers, durch Drücken auf den Verbundkörper erfolgen, wobei der Wirkstoff durch den jeweiligen Medienkanal und die zugeordnete Applikationsöffnung herausgedrückt wird.

Vorteilhafterweise ist dabei die Wirkstoffkammer zum zugeordneten Medienkanal hin durch ein Siegel- oder Abschlusselement verschlossen, so dass ein unbeabsichtigtes Austreten des Wirkstoffs vermieden ist. Das Siegelelement ist dabei vorteilhafterweise derart ausgelegt, dass es zerreißt, zerbricht oder auf sonstige Weise zerstört wird, wenn Druck auf die Wirkstoffkammer ausgeübt wird, oder es von der Applikationsdüse separiert wird. Vorzugsweise sind dort die Folienelemente laminiert oder verschweißt und durch eine Prägung extra dünn gestaltet, sodass sich eine Schwachstelle ergibt, welche eine Separier- oder Abrissstelle / - bereich bildet und damit die Medienkanäle geöffnet werden. Damit wird infolge des ausgeübten Drucks oder des Separierens der Medienkanal für den Wirkstoff freigegeben, und dieser kann ausgebracht werden.

Durch die genannte Ausgestaltung könnte auch die Logistik für die Bereitstellung von Wirkstoffen insgesamt weiter verbessert werden, da für manche Wirkstoffe oder Medikamente die Selbstverabreichung durch den Patienten überhaupt erst möglich gemacht würde.

In einer besonders bevorzugten, ebenfalls als eigenständig erfinderisch angesehenen Ausgestaltung ist in einer der genannten Wirkstoffkammern als Wirkstoff ein Hydrogencarbonat, bevorzugt Kalium-, Natrium- oder Calciumhydrogencarbonat, besonders bevorzugt Natriumhydrogencarbonat, vorgehalten. Mit einer solchermaßen präparierten und vorbefüllten Applikationsdüse könnte der Patient oder Nutzer beispielsweise im Zuge einer so genannten Parodontosebehandlung oder deren Nachsorge den Behandlungserfolg eigenständig überwachen und nachverfolgen.

Falls nämlich das Carbonat mittels der Applikationsdüse in die befallene Zahntasche appliziert wird und dort eine Infektion vorliegt, wechselwirkt das Carbonat mit der durch die Infektion bedingt sauren Umgebung in der Zahntasche unter Bläschenbildung. Dieser Effekt der Bläschenbildung in der Tasche kann einerseits für den Nutzer als Indikator genutzt werden: das Sprudeln findet nämlich nur statt, wenn die Umgebung sauer ist, und somit eine Infektion in der Tasche vorliegt. Dadurch bekommt der Nutzer durch das Sprudeln somit sowohl ein haptisches oder fühlbares als auch ein optisches Feedback über das Vorliegen einer Infektion und somit den Fortschritt des Behandlungserfolgs. Zudem bewirkt die Bläschenbildung in der Tasche auch noch einen zusätzlichen Spülerfolg und begünstigt damit auch die Nachsorge der Behandlung.

Ein solches Konzept, nämlich die Bereitstellung eines Carbonats oder Hydrogencarbonats als Wirkstoff in einer über einen Medienkanal mit einer Applikationsöffnung verbundenen Wirkstoffkammer, und ein solchermaßen aufgebauter Wirkstoffapplikator werden als eigenständig erfinderisch angesehen, auch (aber nicht nur) in einer Ausgestaltung mit lediglich einem Medienkanal und/oder lediglich einer Wirkstoffkammer, also insbesondere ohne die Bereitstellung eines weiteren Wirkstoffs, und bevorzugt in Kombination mit den vorstehend genannten Aspekten bezüglich der räumlichen und/oder funktionalen Ausgestaltung der Applikatorkomponenten. Gemäß einem als eigenständig erfinderisch angesehenen Aspekt der Erfindung kann eine solche Diagnose über die Anwesenheit einer Entzündung auch erfolgen, indem eine Papierspitze, eine Wattespitze, ein flüssigkeitssammelndes Silikonelement oder dergleichen in die Zahntasche eingebracht und dort eine Probe entnommen wird, die dann in eine Lösung mit Na- oder K-Hydrogencarbonat gegeben wird. Somit kann auf einfache Weise ein Indikator für die Feststellung einer Entzündung bereitgestellt werden, die für die Benutzung durch den Patienten für zu Hause geeignet ist.

Gemäß dem vorstehend genannten Aspekt der Erfindung sind zwei oder mehrere Wirkstoffkammern vorgesehen, von denen jede über jeweils einen zugeordneten Medienkanal mit jeweils mindestens einer Applikationsöffnung verbunden ist. In den Kammern können dann gemäß einem Aspekt der Erfindung unterschiedliche Wirkstoffe vorgehalten sein, die in Kombination oder Wechselwirkung miteinander die erwünschten Effekte auslösen. Die Wirkstoffe werden dann bei der Betätigung durch den Benutzer gemeinsam ausgebracht und vermischen sich am Applikationsort, so dass die erwartete Wechselwirkung am Applikationsort ausgelöst wird. Beispielsweise könnten zwei Wirkstoffkammern vorgesehen sein, wobei in einer das Hydrogencarbonat und in der anderen eine Säure / Carbonsäure (z. B. Äpfelsäure, Zitronensäure, Milchsäure etc.) vorgehalten wird, vorzugsweise jeweils in wässriger Lösung und in für die angestrebte Handhabung geeigneter Konzentration. Diese werden dann gemeinsam ausgebracht, kommen am Applikationsort in Kontakt miteinander und reagieren unter Bläschenbildung. Dieser Effekt könnte beispielsweise zu Reinigungs- oder Spülzwecken besonders günstig eingesetzt werden.

Als Wirkstoff kann eine der Wirkstoffkammern insbesondere mit Dinatriumhydrogenphosphat, Natriumdihydrogenphosphat und/oder Natriummonohydrogenphosphat, jeweils ggf. auch als Dihydrat, befüllt sein. Alternativ oder zusätzlich kann gemäß einem Aspekt der Erfindung auch ein aufweichender Stoff, beispielsweise Docusat-Natrium, und/oder ein antibakterieller Stoff mit enthalten sein, wie beispielsweise Nelkenöl, CHX oder H₂O₂.

Die mit der Erfindung erzielten Vorteile bestehen insbesondere darin, dass durch die Bereitstellung zweier oder mehrerer Wirkstoffe in separat gehaltenen Wirkstoffkammern, die über separate Medienkanäle in einem gemeinsamen Raumbereich ausgebacht und dort zur Vermischung gebracht werden, die gezielte und lokalisierte Nutzung der Reaktionsdynamik der Wirkstoffe und/oder deren Reaktionsprodukte, wie beispielsweise gebildete Gasbläschen, ermöglicht wird. Gerade bei der Ausbringung in den ansonsten eigentlich nur schwer zugänglichen Zahntaschen können dort besondere Pflege- oder Behandlungsmaßnahmen durchgeführt werden, selbst durch den Nutzer oder Patienten selbst, und ohne medizinische Vorbildung. Die Ausgestaltung der Applikationsdüse als Folien-Verbundkörper oder Laminat ermöglicht zudem, dass mit vergleichsweise einfachen Mitteln und unter enormer Flexibilität bei der räumlichen Gestaltung kostengünstig und in großen Stückzahlen geeignete Applikationsdüsen bereitgestellt werden können. Auch damit wird die Versorgung in einer Vielzahl von Anwendungsfällen, ausgehend von therapeutischen Anwendungen durch geschultes Personal bis hin zu Vorsorge- oder Pflegemaßnahmen, die der Nutzer oder Patient selbst durchführen kann, ermöglicht oder zumindest begünstigt.

Darüber hinaus ist noch zu berücksichtigen, dass ein Anwender zu Hause keine sterilen Medien, insbesondere Gase, zur Verfügung hat. Im Gegensatz dazu hat ein Zahnarzt meist sterile Luft und steriles Wasser zum Spülen der Zahntaschen im Zugriff. Durch die nunmehr vorgesehene Mischung von mindestens zwei sterilen Wirkstoffen mit vergleichsweise kleinem Volumen entsteht unmittelbar vor Ort, also bereits in der Zahntasche, als Reaktionsprodukt ein steriles Gas mit sehr hohem Volumen, wodurch eine sterile Spülung der Zahntasche selbst für den Privatanwender ermöglicht wird. Dies ist insbesondere bei der Spülung der Zahntasche durch den Patienten selbst von großer Bedeutung. Weiterhin kann durch das erfindungsgemäße Konzept ein steriles Gas zum Ausspülen direkt am Einsatzort bereitgestellt werden.

Durch das Einbringen einer Hydrogencarbonatlösung in die Zahntasche wird neben dem Erkennen einer entzündeten Tasche auch der pH-Wert innerhalb der Tasche angehoben. Insbesondere die Zahnsubstanz und das den Zahn umgebende Hart- und Weichgewebe reagieren nämlich entzündlich auf eine durch einen Biofilm bedingte pH-Wert Senkung innerhalb der Zahntasche. Folglich besteht die Möglichkeit, das Milieu innerhalb der Zahntasche, der Zahnsubstanz und/oder dem den Zahn umgebenden Hart- und Weichgewebe günstiger zu gestalten und einen höheren pH-Wert einzustellen, was das Gewebe regenerativ unterstützt.

Die Reaktion von in Wasser gelösten Hydrogencarbonaten wie z.B. Kalium- und oder Natriumhydrogencarbonat mit Säuren (z.B. in Wasser gelöste Äpfelsäure, Zitronensäure oder Milchsäure) bzw. den H+-Ionen von Säuren besteht darin, dass sich das Gas Kohlendioxid (CO₂), Wasser (H2O) und das Natriumsalz des Säureanions (im Beispiel Natriummalat - Na₂C₄H₄O₅, Natriumcitrat - Na₃C₆H₅O₇ bzw. Natriumlactat - NaC₃H₅O₃) bilden. Als besonders vorteilhaft und eigenständig erfinderisch im Hinblick auf die nunmehr vorgesehenen Substanzen wird angesehen, dass bei dieser chemischen Reaktion neben der spülenden und gasblasenbildenden Kohlendioxid-Bildung keinerlei toxischen oder medizinisch bedenklichen Stoffe entstehen.

So wäre das Ammoniumhydrogencarbonat an sich zwar eine denkbare Alternative, aber dieses selbst ist bereits gesundheitsschädlich. Auch würden bei der Reaktion mit einer Säure oder Carbonsäure gesundheitsschädliche Stoffe entstehen. Bei der Reaktion mit einer Säure oder Carbonsäure bilden sich Wasser (H2O), das vorteilhafte und gewünschte Kohlendioxid (CO2) und das gesundheitsschädliche und giftige Ammoniak (NH3).

Als einzig echte Alternative neben dem Kalium- und Natriumhydrogencarbonat wird somit das ausschließlich in Wasser existente Calciumhydrogencarbonat angenommen.

Diese Art der Spülung lässt sich natürlich auch in vielen anderen medizinischen Anwendung am menschlichen oder tierischen Körper, bedingt durch die hohe Sterilität des entstehenden Gases anwenden.

Ein Ausführungsbeispiel der Erfindung wird anhand einer Zeichnung näher erläutert. Darin zeigen:
- FIG. 1: schematisch im Längsschnitt einen Zahn im Mundknochen eines Patienten,
- FIG. 2: schematisch einen Wirkstoffapplikator in mehrteiliger Ausführung,
- FIG. 3: eine Applikationsdüse des Wirkstoffapplikators gem. FIG. 2 in perspektivischer Ansicht,
- FIG. 4: einen einteilig ausgeführten Wirkstoffapplikator in perspektivischer Ansicht,
- FIG. 5: eine Applikationsdüse des Wirkstoffapplikators nach FIG. 2 oder FIG. 3 in perspektivischer Ansicht des Behandlungsendes sowie im Querschnitt,
- FIG. 6: ein Basalsegment der Applikationsdüse gem. FIG. 3 in perspektivischer Ansicht in einer Sequenz von Herstellungsschritten,
- FIG. 7: ein Basalsegment des Wirkstoffapplikators gem. FIG. 4,
- FIG. 8: eine ausschnittsweise Vergrößerung von FIG. 7,
- FIG. 9: ein Basalsegment des Wirkstoffapplikators gem. FIG. 4 mit alternativer Kanalgeometrie im Bereich der Mischstelle,
- FIG. 10: eine ausschnittsweise Vergrößerung von FIG. 9,
- FIG. 11: ein Basalsegment des Wirkstoffapplikators gem. FIG. 4 mit weiterer alternativer Kanalgeometrie im Bereich der Mischstelle,
- FIG. 12: eine ausschnittsweise Vergrößerung von FIG. 11,
- FIG. 13: ein Basalsegment des Wirkstoffapplikators gem. FIG. 4 mit weiterer alternativer Kanalgeometrie im Bereich der Mischstelle,
- FIG. 14: eine ausschnittsweise Vergrößerung von FIG. 13,
- FIG. 15: ein Basalsegment des Wirkstoffapplikators gem. FIG. 4 mit weiterer alternativer Kanalgeometrie im Bereich der Mischstelle,
- FIG. 16: eine ausschnittsweise Vergrößerung von FIG. 15,
- FIG. 17: eine Variante des Wirkstoffapplikators gem. FIG. 4 in perspektivischer Ansicht in unterschiedlichen Teilschnitten,
- FIG. 18: eine weitere Variante des Wirkstoffapplikators gem. FIG. 4 in perspektivischer Ansicht in unterschiedlichen Teilschnitten,
- FIG. 19: eine weitere Variante des Wirkstoffapplikators gem. FIG. 4 in perspektivischer Ansicht einer Sequenz von Herstellungsschritten,
- FIG. 20: ein Zwischenstück in perspektivischer Ansicht in einer Sequenz von Herstellungsschritten,
- FIG. 21: das Zwischenstück gem. FIG. 20 mit angeschlossenen Medienleitungen,
- FIG. 22: ein Doppelspritzensystem in unterschiedlichen Ansichten,
- FIG.23: ein Schlauchpaket.
- FIG. 24: eine alternative Ausführungsform eines Wirkstoffapplikators,
- FIG. 25: den Bodenbereich zweier Wirkstoffkammern im Längsschnitt,
- FIG. 26: eine Applikationsdüse im Längsschnitt,
- FIG. 27: eine alternative Applikationsdüse im Längsschnitt,
- FIG. 28: die Applikationsdüse gem. FIG. 27 im vergrößerten Ausschnitt,
- FIG. 29: den Wirkstoffapplikator gem. FIG. 24 mit zugeordnetem Siegelsystem,
- FIG. 30: den Verschlussbereich des Wirkstoffapplikators gem. FIG. 29 in vergrößerter Darstellung,
- FIG. 31: einen Wirkstoffapplikator mit abgeschrägter Applikationsdüse, und
- FIG. 32, 33: jeweils weitere alternativ ausgeführte Wirkstoffapplikatoren.

Gleiche Teile sind in allen Figuren mit denselben Bezugszeichen versehen.

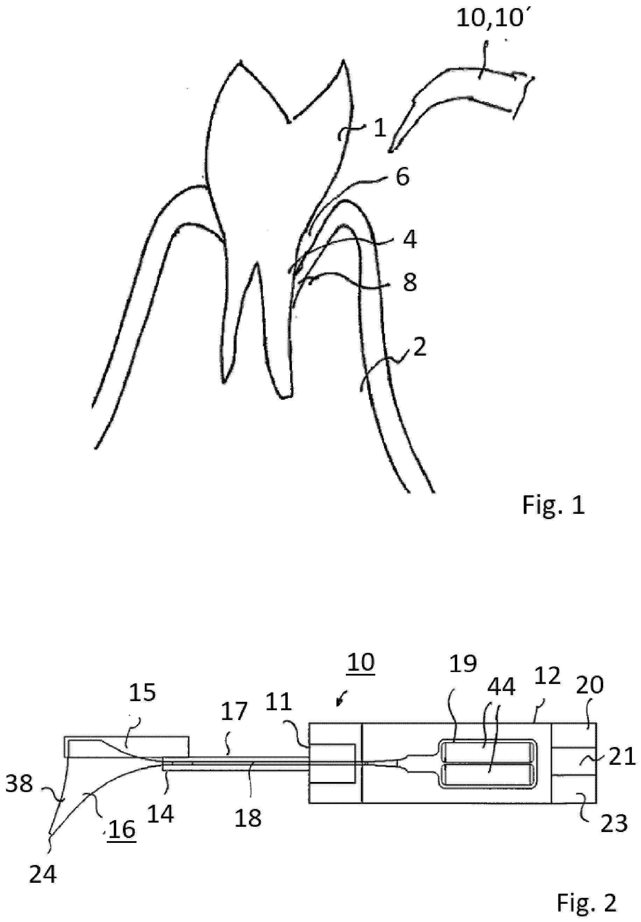

**In** FIG. 1 ist schematisch im Längsschnitt ein Zahn 1 im Kieferknochen 2 eines Patienten gezeigt. Dieser Zahn 1, der lediglich beispielhaft zur Darstellung der Wirkungsweise der vorliegenden Erfindung dient, ist im Ausführungsbeispiel im Status nach einer durchgeführten Parodontosebehandlung gezeigt, wobei sich in der Nachbarschaft zum Zahnhals 4 eine vergleichsweise tiefe Zahntasche 6 gebildet hat. Diese Zahntasche 6 hat sich infolge der Parodontose, durch ein Eindringen von Bakterien oder Keimen in den Gewebebereich, insbesondere im Bereich des Zahnhalses 4 und im umgebenden Weichgewebe 8, entzündet. Daher ist nach der eigentlichen therapeutischen Behandlung der Parodontose in der Art einer Nachsorge eine nachgelagerte Behandlung vorgesehen, die gegebenenfalls vom Patienten selbst von zu Hause aus, beispielsweise auf täglicher Basis, durchgeführt werden soll. Bei dieser Behandlung ist vorgesehen, zielgenau einen dentalen Wirkstoff, beispielsweise ein Desinfektionsmittel zur Abtötung der vorhandenen Bakterien oder Keime oder auch eine Spüllösung, im Mundraum des Patienten in die Zahntasche 6 und damit den befallenen Bereich im umgebenden Weichgewebe auszubringen.

Zu diesem Zweck ist ein in FIG. 1 in räumlicher Nähe zur Zahntasche 6 und in FIG. 2 vergrößert in seitlicher Ansicht dargestellter Wirkstoffapplikator 10, 10' vorgesehen, der dem Patienten bereitgestellt wird, so dass dieser selbst und von zu Hause aus die jeweiligen Behandlungen durchführen und den vorgesehenen Wirkstoff zielgenau im Mundraum ausbringen kann. Es wird darauf verwiesen, dass Aufbau und Funktionsweise des Wirkstoffapplikators 10, 10' vorliegend lediglich beispielhaft anhand der Ausbringung von Wirkstoff in die Zahntasche 6 beschrieben werden; es sind selbstverständlich aber noch vielfältige andere Nutzungsarten, beispielsweise im Rahmen einer (nicht-therapeutischen) Zahnpflege oder -reinigung, zur Reinigung von Zahnzwischenräumen, zur gezielten Ausbringung von Wirkstoffen an lokale Entzündungen im Bereich der Mundhöhle oder ähnliches möglich und im Rahmen des vorliegenden Erfindungsgedankens mit erfasst.

Der in FIG. 2 dargestellte Wirkstoffapplikator 10 ist im Ausführungsbeispiel als mobiles mehrteiliges System ausgeführt. Er umfasst ein in der Art eines Gehäuses ausgestaltetes Hand- oder Griffstück 12, das an seinem freien oder distale Ende 14 als eigentlichen Behandlungskopf oder eigentliches Behandlungselement eine Applikationsdüse 16 aufweist, die im Ausführungsbeispiel im Hinblick auf Hygiene- und Sorgfaltserwägungen für eine lediglich einmalige Verwendung und somit als Einmal- oder Wegwerfprodukt ausgeführt ist. Die Applikationsdüse 16 ist über Verbindungselemente, im Ausführungsbeispiel ein in einer Schutzhülle 17 geführtes Schlauchstück 18, mit einem im Griffstück 12 angeordneten Versorgungscontainer 19 verbunden, in dem die vorgesehenen Wirkstoffe vorgehalten sind. Das Schlauchstück 18 mitsamt der Schutzhülle 17 ist dabei über eine mit einem Schlauchventil 11 absperrbare Durchführung aus dem vom Griffstück 12 gebildeten Gehäuse herausgeführt.

Weiterhin sind im Griffstück 12 ein System zur Betätigung des Wirkstoffcontainers zur automatisierten Ausbringung von Wirkstoff, beispielsweise ein mechanisches, pneumatisches oder hydraulisches Drucksystem 23, mit dem der oder die Wirkstoffe herausgefördert werden können, sowie eine Steuereinheit 20 und eine Stromversorgungseinheit 21, beispielsweise eine Batterie oder ein Akku, angeordnet. Als mechanisches System könnte dabei beispielsweise ein auf den Wirkstoffcontainer wirkender Druckstempel oder auch dessen Ausgestaltung ähnlich einer Doppelspritze oder Doppelkanüle bzw. Doppelkarpulensystem vorgesehen sein. Dem Grunde nach ähnelt der Wirkstoffapplikator 10 vom Grundsatz her somit Art und Aufbau einer elektrischen Zahnbürste, deren aktive (elektrische) Komponenten ebenfalls im das Außengehäuse bildenden Griffstück integriert sind. Die Applikationsdüse 16 ist im Ausführungsbeispiel an dem Fixierungsstück 15 befestigt. Alternativ könnte der Wirkstoffapplikator 10 aber auch als ortsfest installiertes Applikationssystem, beispielsweise in den Behandlungsräumen eines Zahnarztes, ausgeführt sein; in diesem Fall könnte das Griffstück beispielsweise seinerseits mehrteilig ausgeführt sein und ein Mundstück aufweisen, das die Applikationsdüse 16 trägt, und das seinerseits an ein Versorgungssystem 19 angeschlossen ist, in dem die Wirkstoffe vorgehalten werden.

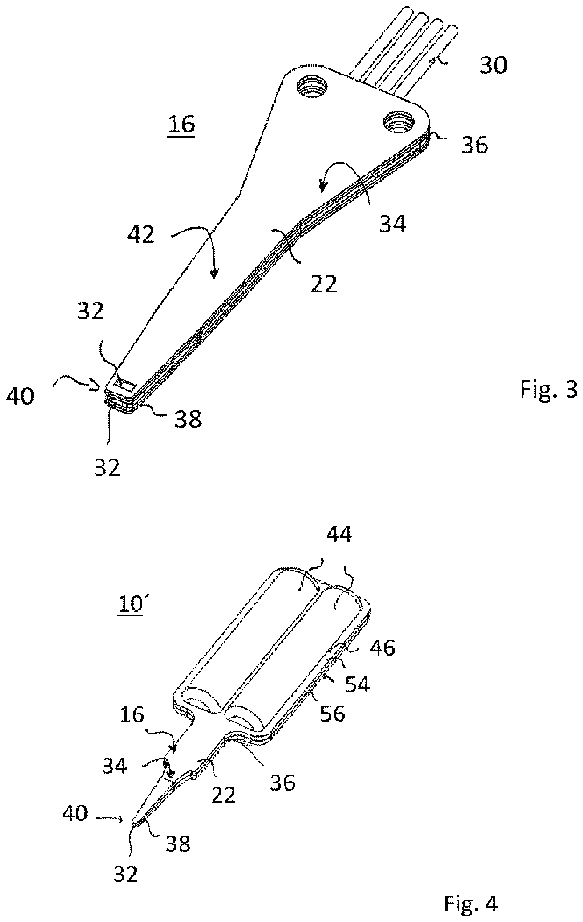

Die Applikationsdüse 16 ist in FIG. 3 in vergrößerter Ansicht perspektivisch in einer Ausführung dargestellt, in der eingangsseitig vier Zugangsstutzen 30 mit jeweils einem innerhalb ihres Düsenkörpers 22 verlaufenden Medienkanal verbunden sind. Alternativ kann selbstverständlich auch eine andere Anzahl solche Zugangsstutzen 30, beispielsweise ein einziger, zwei oder mehrere, vorgesehen sein. Über diese Zugangsstutzen 30 kann die Applikationsdüse 16 beispielsweise mit dem Schlauchpaket 18 verbunden sein.

Eine alternative, einstückige Ausführung des Wirkstoffapplikators 10' ist in FIG. 4 in perspektivischer Ansicht gezeigt. Bei dieser Ausführungsform ist die Applikationsdüse 16 integrierter Teil des Wirkstoffapplikators 10' und an diesen angeformt. Im Hinblick auf die vorgesehene bevorzugte Verwendung im Bereich der Zahnpflege oder als Behandlungsmittel auch für den Selbstgebrauch ist die Applikationsdüse 16 bei beiden Ausführungsformen gem. FIG. 2, 3 und 4 gezielt für eine hohe Funktionalität bei besonders einfach gehaltener Bauweise ausgeführt, so dass bei nur begrenzten Herstellungskosten eine Herstellung auch in enorm hohen Stückzahlen ermöglicht ist. Dazu weist die Applikationsdüse 16 als wesentliche Funktionskomponente den Düsenkörper 22 auf, in dem eine Anzahl von auslassseitig ggf. in jeweils einer Applikationsöffnung 32 mündenden Medienkanälen 34 für den zu applizierenden Wirkstoff angeordnet sind.

Wie den perspektivischen Darstellungen in FIG. 3 und 4 gut entnehmbar ist, ist der Düsenkörper 22 als sich in einer Längsrichtung von einem Anschlussbereich 36 aus hin zu einem freien Behandlungsende 38 hin erstreckender Körper ausgeführt. In der einstückigen Ausführung gem. FIG. 4 liegt der Anschlussbereich 36 dabei innerhalb des Applikatorkörpers; in der für die mehrteilige Variante vorgesehenen Ausführung gem. FIG. 3 entspricht der Anschlussbereich 36 hingegen funktional der Anschlussseite der Applikationsdüse 16. Diese Anschlussseite ist dabei im Ausführungsbeispiel mit geeigneten Mitteln zur mechanischen Befestigung des Düsenkörpers 22 am Griffstück 12 versehen und mit dem Schlauchstück 18 verbunden; in der alternativ vorgesehenen eigenständigen Ausgestaltung gem. FIG. 4 ist ein derartiger Anschluss hingegen nicht erforderlich.

Bei der Formgebung des Düsenkörpers 22 ist insbesondere dem Umstand Rechnung getragen, dass der Nutzer trotz sehr beengter Raumverhältnisse in der Mundhöhle das Behandlungsende 38 sehr zielgenau positionieren können sollte, um den Wirkstoff genau lokalisiert ausbringen zu können. Um dies zu ermöglichen, ist die Raumform des Düsenkörpers 22 unter Berücksichtigung des Umstands geeignet gewählt, dass die Zahntasche 6 sich üblicherweise in der Art eines sich entlang der Zahnoberfläche erstreckenden Spalts ausbildet.

Um dem Rechnung zu tragen, ist gemäß einem Aspekt der Erfindung die Ausführung des Düsenkörpers 22 der Applikationsdüse 16 in der Art eines flach gehaltenen Raumkörpers als im wesentlichen flächiges Bauteil vorgesehen. Die Applikationsdüse 16 bzw. der diese bildende Grundkörper 22 ist somit als im Wesentlichen entlang einer basalen Ebene oder Grundebene ausgedehnter Körper ausgeführt, dessen Dicke im Querschnitt gesehen deutlich geringer gehalten ist als seine laterale Ausdehnung in der Basalebene. Für die Anwendung bedeutet dies, dass das freie oder Behandlungsende 38 des Düsenkörpers 22 vergleichsweise einfach in die Zahntaschen 6 einbringbar ist, indem die Basalebene des Düsenkörpers 22 beispielsweise im Wesentlichen parallel zur Außenoberfläche des Zahns 1 ausgerichtet wird.

Des Weiteren verjüngt sich der Grundkörper 22 in seinem Querschnitt in Richtung auf das Behandlungsende 38 hin. Das freie oder Behandlungsende 38 des Düsenkörpers 22 weist somit im Wesentlichen eine flache, vergleichsweise schmal oder sogar spitz zulaufende Kontur auf, so dass die Einbringung in die Zahntaschen 6 besonders einfach möglich ist. Im Ausführungsbeispiel resultiert dies, wie beispielsweise den perspektivischen Darstellungen gem. FIG. 3, 4 entnehmbar ist, darin, dass der Düsenkörper 22 in Draufsicht eine in der Art eines Dreiecks geformte Kontur aufweist.

Um eine zielgenaue Ausbringung des Wirkstoffs in die Zahntasche 6 zu ermöglichen, ist der Düsenkörper 22 in einem im Bereich des Behandlungsendes 38 angeordneten Ausströmbereich 40 mit den jeweils mit einem der Medienkanäle 34 verbundenen Applikationsöffnungen 32 für den jeweiligen Wirkstoff versehen. Damit kann der jeweilige Wirkstoff gezielt im Bereich des Behandlungsendes 38 der Applikationsdüse 16 und somit im Bedarfsfall direkt in die jeweilige Zahntasche 6 ausgebracht werden. Eine Anzahl der Applikationsöffnungen 32 sind dabei zudem in einer seitlich zur Längsachse ausgerichteten Abströmrichtung angeordnet, wie beispielsweise durch die perspektivischen Darstellungen in FIG. 3, 4 ersichtlich wird. Damit kann auf einfache Weise der vollständige Raumbereich in der Zahntasche 6 rund um das Behandlungsende 38 des Düsenkörpers 22 herum mit Wirkstoff geflutet werden.

Hinsichtlich der Führung der Medienkanäle 34 im Düsenkörper 22 sind im Rahmen der vorliegenden Erfindung grundsätzlich zwei bevorzugte Auslegungskonzepte vorgesehen, die jeweils unabhängig voneinander als eigenständig erfinderisch angesehen werden.

Gemäß dem ersten dieser Auslegungskonzepte ist in beiden Ausführungsformen gem. FIG. 2 und FIG. 4 der Wirkstoffapplikator 10, 10', jeweils durch geeignete Ausgestaltung der Applikationsdüse 16, gemäß einem Aspekt der Erfindung für das Konzept ausgelegt, dass zwei oder mehr Wirkstoffe separat und getrennt voneinander am Applikationsort ausgebracht und dort miteinander vermischt und zur Reaktion gebracht werden, so dass die Reaktion an sich und/oder die Reaktionsprodukte am Ausbringungsort eine Wirkung entfalten können. Dazu sind in den Düsenkörper 22 mindestens zwei unabhängig voneinander vom Anschlussbereich 36 zum freien Behandlungsende 38 hin verlaufende, auslassseitig in jeweils einer Applikationsöffnung 32 mündende Medienkanäle 34 integriert. Die den beiden Medienkanälen 34 zugeordneten Applikationsöffnungen 32 sind in den gezeigten Ausführungsbeispielen jeweils etwa 1 mm voneinander beabstandet, so dass die Ausbringung der Wirkstoffe ausreichend nah zueinander am Applikationsort erfolgen kann, um die angestrebte lokale Durchmischung zu ermöglich.

Gemäß dem zweiten, als eigenständig erfinderisch angesehenen Auslegungskonzept ist hingegen in beiden Ausführungsformen gem. FIG. 2 und FIG. 4 der Wirkstoffapplikator 10, 10', jeweils durch geeignete Ausgestaltung der Applikationsdüse 16, gemäß einem Aspekt der Erfindung für das Konzept ausgelegt, dass zwei oder mehr Wirkstoffe dem Düsenkörper 22 separat und getrennt voneinander zugeführt und sodann an einer Mischstelle 42 miteinander vermischt und somit ggf. zur Reaktion miteinander gebracht werden. Ausgehend von der Mischstelle 42 führt dann ein weiterer Medienkanal 34 zum freien Behandlungsende 38, so dass die vermischten Medien am Applikationsort ausgebracht werden. Dazu sind in den Düsenkörper 22 mindestens zwei unabhängig voneinander vom Anschlussbereich 36 zur Mischstelle 42 führende Medienkanäle 34 sowie ein weiterer, zum freien Behandlungsende 38 hin verlaufender, auslassseitig in einer Applikationsöffnung 32 mündender Medienkanal 34 integriert. Darüber hinaus können auch noch weitere, beispielsweise direkt vom Anschlussbereich 36 zum freien Behandlungsende 38 führende Medienkanäle 34 vorgesehen sein, so dass weitere Medien auch direkt durch den Düsenkörper 22 hindurch zum freien Behandlungsende 38 hin befördert werden können, beispielsweise gemäß dem vorstehend beschriebenen ersten Auslegungskonzept. Die diesen Medienkanälen 34 zugeordneten Applikationsöffnungen 32 sind in den gezeigten Ausführungsbeispielen jeweils etwa 1 mm voneinander beabstandet, so dass die Ausbringung der Wirkstoffe ausreichend nah zueinander am Applikationsort erfolgen kann, um möglicherweise eine angestrebte lokale Durchmischung zu ermöglich.

Jeder vom Anschlussbereich 36 ausgehenden Medienkanäle 34 ist in beiden Auslegungskonzepten eingangsseitig, beispielsweise über den jeweiligen Zugangsstutzen 30, medienseitig jeweils mit einer separat zugeordneten Wirkstoffkammer 44 verbunden. Die jeweilige Wirk-stoffkammer 44 weist dabei gemäß einem Aspekt der Erfindung ein an die zu verabreichende Patientendosis angepasstes Innenvolumen auf, so dass für den Anwender oder Nutzer keine weitere Dosierung notwendig ist.

Beim Wirkstoffapplikator 10 gem. FIG. 2 sind die Wirkstoffkammern 44 in den Versorgungscontainer integriert, und die den Wirkstoffkammern 44 jeweils individuell zugeordneten Medienkanäle 34 sind innerhalb des Schlauchstücks 18 bis hin zum Anschlussbereich 36 geführt. Wenn die Wirkstoffkammern 44 in dieser Ausführung mit Druck beaufschlagt werden, wird somit der enthaltene Wirkstoff über die Medienkanäle 34 und die Applikationsdüse 16 zum Ausströmbereich 40 transportiert und dort über die Applikationsöffnungen 32 ausgebracht. Demgegenüber sind die Wirkstoffkammern 44 beim Wirkstoffapplikator 10' gem. FIG. 4 in einen den Düsenkörper 22 umfassenden gemeinsamen Grundkörper 46 integriert.

Im gezeigten Ausführungsbeispiel sind gemäß eines als eigenständig erfinderisch angesehenen Aspekts jeweils zwei Wirkstoffkammern 44 vorgesehen, in denen für die angestrebte Wirkung geeignete, unterschiedliche Wirkstoffe vorgehalten sind, die in Kombination oder Wechselwirkung miteinander die erwünschten Effekte auslösen. Beispielsweise könnten zwei Wirkstoffkammern 44 vorgesehen sein, wobei in einer ein Carbonat, insbesondere ein Hydrogencarbonat, und in der anderen eine Säure (z. B. Zitronensäure) vorgehalten wird. Diese werden dann gemeinsam ausgebracht, kommen an der Mischstelle 42 in Kontakt miteinander und reagieren dort unter Gas- und Bläschenbildung. Damit kann in der Mischstelle 42 gezielt ein unter vergleichsweise hohem Druck stehendes Gas gebildet werden, das dann über den der Mischstelle 42 nachgeschalteten Medienkanal 34 am Behandlungsende 38 ausgebracht wird. Dieser Effekt könnte beispielsweise zu Reinigungs- oder Spülzwecken besonders günstig eingesetzt werden, wobei das unter hohem Druck austretende Gas einen besonders guten Reinigungseffekt erzielt.

Alternativ oder zusätzlich kann in einer solchen Wirkstoffkammer 44, die nach der Befüllung zum benachbarten Medienkanal 34 mit einem Siegel- oder Verschlusselement verschlossen ist, beispielsweise eine vordefinierte Dosis an Wirkstoff, z. B. Chlorhexidin zur Desinfektion oder eine Spüllösung, enthalten sein. Der Patient kann diese dann selbst zu Hause applizieren, wobei einerseits durch die Bauart der Applikationsdüse 16 bedingt eine korrekte Positionierung im Mundraum ermöglicht wird und andererseits durch die portionierte Vorhaltung im Düsenkörper die korrekte Dosierung des Wirkstoffs sichergestellt werden kann.

Weiterhin kann gemäß einem Aspekt der Erfindung im Düsenkörper 22 zusätzlich zu einem auslassseitig in eine Applikationsöffnung 32 mündenden, als Applikations-Medienkanal vorgesehenen Medienkanal 34 mindestens einer der Medienkanäle 34 als Absaugkanal ausgebildet sein. Eine solche Ausgestaltung kommt insbesondere bei der in FIG. 3 gezeigten Ausführungsform mit vier Zugangsstutzen 30 und dementsprechend vier im Düsenkörper 22 verlaufenden Medienkanälen in Betracht.

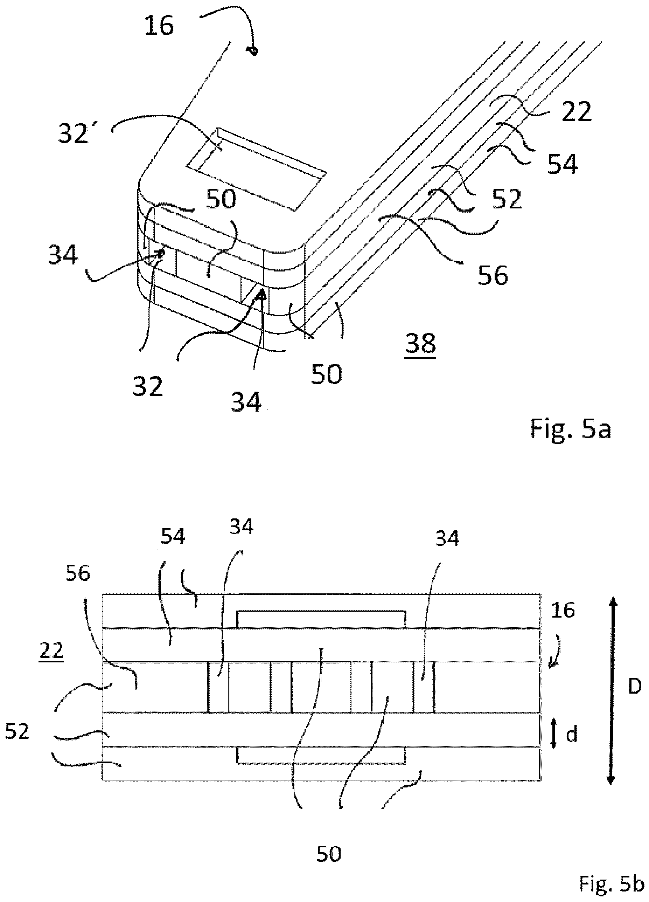

Im Hinblick auf die erwünschte kostengünstige, für hohe Stückzahlen geeignete Bauweise ist gemäß einem Aspekt der Erfindung der Düsenkörper 22, wie in der Darstellung des Behandlungsendes 38 in perspektivischer Ansicht gem. FIG. 5a und im Querschnitt gem. FIG. 5b besonders deutlich erkennbar, in der Art eines Laminatkörpers als aus einer Mehrzahl von Folienstücken 50 aufgebauter Schichtenkörper ausgestaltet. In FIG. 5a ist dabei das Behandlungsende 38 in perspektivischer Ansicht gezeigt; deutlich erkennbar ist dabei, dass einerseits eine Applikationsöffnung 32 in im wesentlichen gerader Abströmrichtung angeordnet ist, wobei zusätzlich auch seitlich ausgerichtete Applikationsöffnungen 32', beispielsweise zur Ausbringung eines weiteren Spülmediums oder auch als Absaugöffnung, vorgesehen sind. Die zugrundeliegende Schichtstruktur ist in der Querschnittsdarstellung gem. FIG. 5b deutlich erkennbar. Die jeweiligen Medienkanäle 34 werden dabei in einer Folienschicht 52 des Laminats durch eine eingebrachte Aussparung in der jeweiligen Schichtfolie gebildet. Durch diese Ausgestaltung der Applikationsdüse 16 bzw. von deren Düsenkörper 22 als Folien-Verbundkörper oder Laminat können mit vergleichsweise einfachen Mitteln und unter enormer Flexibilität bei der räumlichen Gestaltung kostengünstig und in großen Stückzahlen geeignete Applikationsdüsen 16 bereitgestellt werden können.

Diese grundsätzliche Auslegung hat gemäß einem Aspekt der Erfindung den erheblichen Vorteil, dass die Medienkanäle 34 auf vergleichsweise einfache Weise und insbesondere mit erheblicher Flexibilität hinsichtlich Kanalführung, Geometrie und dergleichen durch gängige Folien-Bearbeitungsverfahren wie Ätzen, Prägen, Lasern oder dergleichen in die jeweiligen Folienschichten 52 eingebracht werden können. Damit ist mit einfachen Mitteln eine besonders große Flexibilität hinsichtlich geometrischer, aber auch funktionaler Ausgestaltung und Auslegung der Medienkanäle 34 im Folienstapel erreichbar. Die Applikationsdüse 16 bzw. ihr Düsenkörper 22 ist dabei in der Art eines Laminats oder Schichtenstapels durch eine Anzahl übereinander angeordneter, an ihren Kontaktflächen miteinander verklebten, verschweißten oder auf sonstige Weise verbundenen Folienstücke 50 gebildet. Die Folienstücke 50 weisen dabei jeweils eine Foliendicke d von etwa 150 - 250 µm und somit innerhalb des bevorzugten Bereichs von 50 - 500 µm auf. Die Applikationsdüse 16 bzw. ihr als Laminat aus den Folienstücken 50 aufgebauter Düsenkörper 22 weist somit eine Gesamtdicke D von etwa 0,7 - 1,2 mm, also innerhalb des bevorzugten Bereichs von 0,3 - 2 mm, auf, so dass die angestrebte Einbringung in die Zahntasche 6 problemlos möglich ist.

Gemäß einem Aspekt der Erfindung ist bei der einstückig ausgeführten, in FIG. 4 dargestellten Applikationsdüse 16 diese Bauweise als Folien-Schicht- oder -Verbundkörper oder als Laminat für den gesamten die Applikationsdüse 16 bildenden Düsenkörper 22 vorgesehen. Die Applikationsdüse 16 ist im Ausführungsbeispiel zudem aus hinsichtlich ihrer Materialwahl und -parameter unterschiedlichen Folienschichten 52, 54, 56 aufgebaut, wobei eine zentrale mittlere Folienschicht 56 eines ersten Folienmaterials beidseitig von jeweils einer Seiten- oder äußeren Folienschicht 52, 54 eines anderen Folienmaterials bedeckt ist. Die Folienschicht 56 und die Folienschichten 52, 54 unterscheiden sich in ihren Materialeigenschaften und sind funktional an unterschiedliche Vorgaben geeignet angepasst. Im Ausführungsbeispiel besteht die zentrale Folienschicht 56 gemäß einem Aspekt der Erfindung aus einem vergleichsweise härteren Folienmaterial, also insbesondere mit vergleichsweise größerer Shore-Härte oder E-Modul, wobei die anderen Folienschichten 52, 54 eher weicher sind. Damit kann die zentrale Folienschicht 56 formgebend in der Art einer Stützstruktur die Kontur oder Raumform des Düsenkörpers 22 festlegen, wohingegen die vergleichsweise weicheren äußeren Folienschichten 52, 54 nachgiebig und verformbar ausgestaltet sein können und damit das Verletzungsrisiko bei einem Kontakt beispielsweise der Mundschleimhaut mit dem Düsenkörper 22 deutlich verringern.

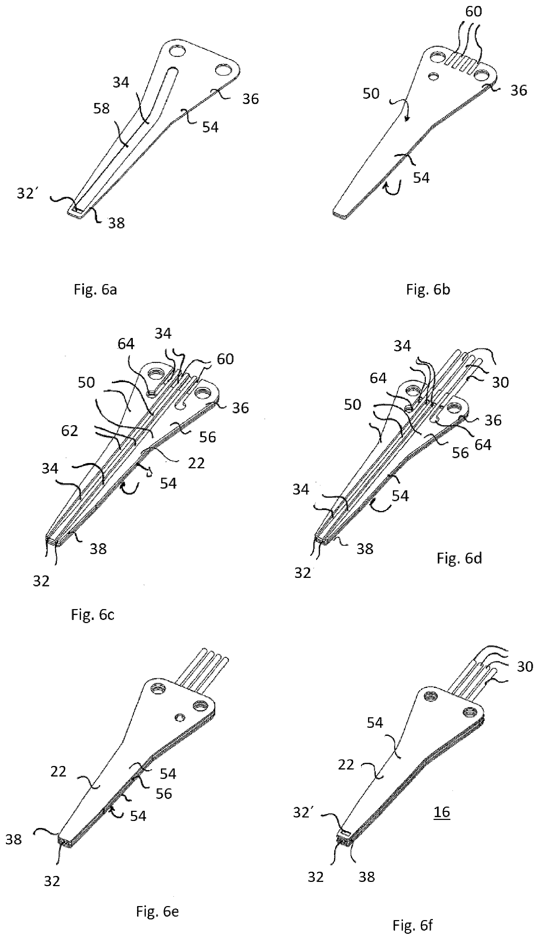

Die in FIG. 3 gezeigte Applikationsdüse 16 besteht im Ausführungsbeispiel in ihrer Gesamtheit aus einem solchen Folienverbund. Diese Bauweise wird aus der Darstellung der schichtweisen Sequenz des Aufbaus in FIG. 6 deutlich.

Der Aufbau des Folienschichtpakets ist in FIG. 6 anhand einer Sequenz, ausgehend von der ersten, untersten Folienschicht 54, unter schrittweiser Hinzufügung der weiteren Folienschichten 54, 56 gezeigt. Dementsprechend zeigt FIG. 6a die unterste oder erste Folienschicht 54, die in Draufsicht gesehen bereits an die gewünschte Form des Düsenkörpers 22 angepasst ist. Ausgehend vom Anschlussbereich 36 verjüngt sich dabei die Breite in Richtung zum Behandlungsende 38 hin. Das solchermaßen in seiner Außenkontur bereits vorgeschnittene Folienstück 54 ist zudem mit einer eingeprägten Nut 58 versehen, die einen Medienkanal 34 bildet. Im gezeigten Ausführungsbeispiel mündet dieser Medienkanal 34 in einer seitlich zur Grundfläche des Düsenkörpers 22 ausgerichteten Applikationsöffnung 32' und ist somit als Kanal beispielsweise für ein Spülmedium besonders geeignet.

Auf diese untere Folienschicht 54 wird anschließend beim schichtweisen Aufbau ein weiteres, ebenfalls eine Folienschicht 54 bildendes Folienstück 50 aufgebracht und beispielsweise auflaminiert. Dieser sich bildende Folienstapel ist in FIG. 6b gezeigt. Dieser umfasst somit zwei übereinanderliegende Folienschichten 54. Die zuletzt aufgebrachte Folienschicht 54 überdeckt dabei den zuvor in die untere Folienschicht 54 eingebrachten, durch die Nut 58 gebildeten Medienkanal 34 und schließt diesen nach oben hin ab. Im Anschlussbereich 36 ist die obere Folienschicht 54 zudem mit Austiefungen 60 versehen, die die Montagepunkte für die spätere Anbringung der Zugangsstutzen 30 vorgeben.

Auf das entstehende Schichtpaket wird sodann, wie in FIG. 6c gezeigt, die nächste Folienschicht 56 aufgebracht. Die Folienschicht 56 bildet dabei die zentrale Folienschicht 56 und ist aus einem vergleichsweise härteren Folienmaterial, also insbesondere mit vergleichsweise größerer Shore-Härte oder E-Modul, ausgeführt, wobei die anderen Folienschichten 54 eher weicher sind. Die Folienschicht 56 kann somit die Funktion einer Stütz- oder formgebenden Schicht übernehmen, die dem gesamten Paket eine gewisse Steifigkeit und mechanische Stabilität gibt.

Deutlich ist in FIG. 6c erkennbar, dass die Folienschicht 56 mehrteilig ausgeführt ist und von einer Anzahl von Folienstücken 50 gebildet wird. Die Folienstücke 50 sind dabei beabstandet voneinander angeordnet, unter Freilassung von dazwischen liegenden Aussparungen 62. Diese Aussparungen 62 bilden ebenfalls einige der in den Düsenkörper 22 integrierten Medienkanäle 34, die aufgrund der Möglichkeiten bei der Bearbeitung der Folien (Lasern, Stanzen) mit großen Freiheitsgraden gestaltet werden können. Im Ausführungsbeispiel gem. FIG. 6c ist dabei deutlich erkennbar, dass, ausgehend vom vorgesehenen Anschlussbereich 36, im dort gezeigten Beispiel vier Medienkanäle 34 in den Düsenkörper 22 hineingeführt sind. Die beiden zentralen der Medienkanäle 34 sind dabei getrennt voneinander vollständig durch den Düsenkörper 22 bis hin zum distalen Behandlungsende 38 hindurchgeführt, so dass mit diesen Kanälen 34 zwei separate Behandlungsmedien unabhängig voneinander ausgebracht werden können. Demgegenüber sind die beiden äußeren Medienkanäle 34 innerhalb der Folienschicht 56 lediglich bis hin zu einem Übertrittspunkt 64 geführt, an dem sie mit weiterführenden Medienkanälen 34 in den benachbarten Folienschichten 54 verbunden sind. Einer dieser beiden Medienkanäle 34 ist dabei beispielsweise mit dem vorstehend bereits beschriebenen Medienkanal 34 in der ersten Folienschicht 54 verbunden.

Nach der Aufbringung der zentralen Folienschicht 56 auf den sich bildenden Folienstapel werden die Zugangsstutzen 30 zum Anschluss externer Medienkanäle oder Zuführleitungen in die Austiefungen 60 eingelegt, wie dies in FIG. 6d gezeigt ist. Anschließend werden zur Vervollständigung der Applikationsdüse 16, analog und symmetrisch zu den in FIG. 6a und 6b gezeigten Folienschichten 54, zwei weitere Folienschichten 54 aufgebracht, wie dies FIGs. 6e und 6f gezeigt ist. Damit ist im gezeigten Ausführungsbeispiel die Applikationsdüse 16 als fünfschichtiges Folienlaminat ausgeführt.

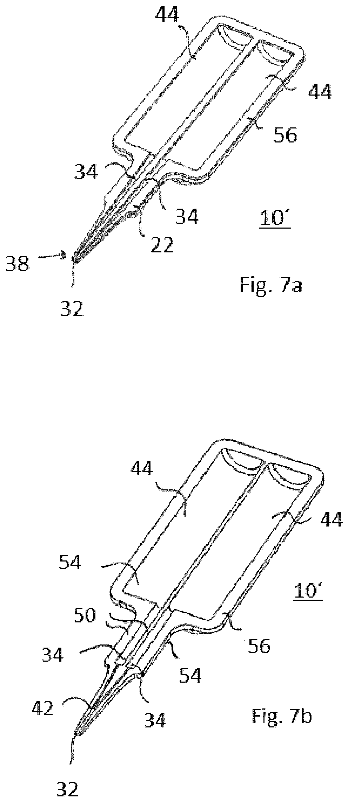

Analog zu dieser Bauweise ist gemäß einem Aspekt der Erfindung auch beim einstückig ausgeführten, in FIG. 4 dargestellten Wirkstoffapplikator 10' diese Bauweise als Folien-Schicht- oder -Verbundkörper oder als Laminat für den gesamten den Wirkstoffapplikator 10' bildenden Grundkörper 46 vorgesehen. Dementsprechend besteht dieser in seiner Gesamtheit aus einem solchen Folienverbund, wobei die Wirkstoffkammern 44 unter Nutzung der Verformbarkeit der Folienschichten 54 direkt in diese eingearbeitet sind. Diese Bauweise wird aus der Darstellung in FIG. 7 deutlich. Dort ist der Wirkstoffapplikator 10' im Umfang seiner "unteren Hälfte" gezeigt, d. h. als Ensemble aus der unteren, mit ihren Ausformungen die Wirkstoffkammern 44 bildenden Folienschicht 54 und der zentralen Folienschicht 56. Die Folienschicht 56 ist dabei wie bereits erläutert geeignet, beispielsweise durch Lasern oder Ätzen, formgebend bearbeitet und bildet die Medienkanäle 34 aus. Die äußeren Folienschichten 54 sind hingegen im hinteren Bereich geeignet ausgeweitet und bilden dort die Wirkstoffkammern 44 aus.

Die vorgesehene Bauweise der Applikationsdüse 16 und das vorgesehene Herstellungsverfahren, insbesondere Lasern oder Einstanzen der Konturen für die Medienkanäle 34, erlaubt eine enorme Flexibilität bei der Auslegung und Konfiguration der im Schichtpaket oder Laminat vorgesehenen Hohlräume, Kavitäten oder Medienvolumina. Die Ausbringung des in der jeweiligen Wirkstoffkammer 44 vorgehaltenen Wirkstoffs kann dann, unter Nutzung der materialbedingt gegebenen Verformbarkeit des Folien-Verbundkörpers, durch Drücken auf den Verbundkörper erfolgen, wobei der Wirkstoff durch den jeweiligen Medienkanal 34 und die zugeordnete Applikationsöffnung 32 herausgedrückt wird. Als Basismaterial für die Folienschichten 52, 54, 56 oder Folienstücke 50 ist Polyamid vorgesehen; alternativ kann aber auch ein anderes geeignetes Folienmaterial wie PP oder PE oder auch eine Kombination unterschiedlicher Folienmaterialien als günstig angesehen werden.

Nachfolgend werden einige als eigenständig erfinderisch angesehene Aspekte der Erfindung weiter erläutert. Diese betreffen weitestgehend die Kanalführung oder weitere Elemente im Bereich der mittleren oder zentralen Folienschicht 56. Sie werden daher anhand einer Darstellung vergleichbar zu FIG. 7, also unter Bezugnahme auf die Ausführungsform des in FIG. 4 gezeigten, einstückigen Wirkstoffapplikators 10' erläutert; selbstverständlich sind diese Aspekte aber ebenso für die in FIG. 3 gezeigte Variante der Applikationsdüse 16 nutzbar.

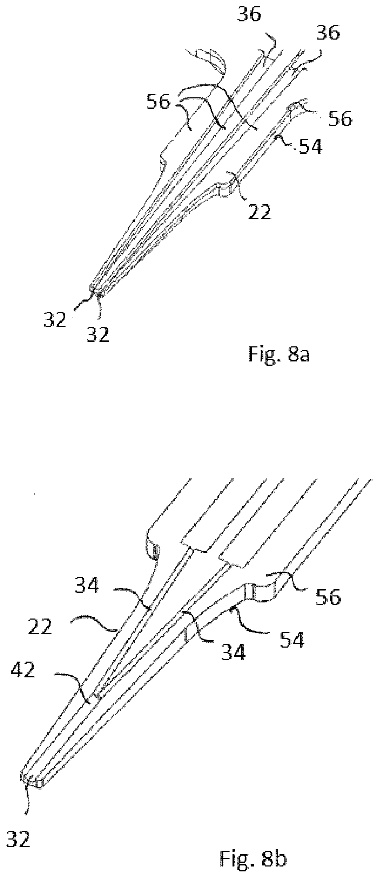

So ist gemäß einem Aspekt der Erfindung und im Sinne des ersten der oben genannten Auslegungskonzepte, wie dies der Darstellung in FIG. 7a und insbesondere der vergrößerten Darstellung in FIG. 8a gut entnehmbar ist, vorgesehen, innerhalb des Folienverbunds in der Folienschicht 56 zwei Medienkanäle 34 als medienseitig voneinander getrennt gehaltene Kanäle anzuordnen, die im Bereich des Behandlungsendes 38 in zwei zwar dicht benachbarten, aber separat und beabstandet voneinander angeordneten Applikationsöffnungen 32 münden.

Dem gegenüber ist gemäß einem weiteren, als eigenständig erfinderisch angesehenen Aspekt der Erfindung, wie dies der Darstellung in FIG. 7b und insbesondere der vergrößerten Darstellung in FIG. 8b gut entnehmbar ist, vorgesehen, innerhalb des Folienverbunds eine Mischstelle 42 für zwei unterschiedliche Wirkstoffe bereitzustellen. Dazu sind in dieser Variante in der Folienschicht 56 zwei Medienkanäle 34 als medienseitig voneinander getrennt gehaltene Zuführkanäle ausgeführt, die in der Mischstelle 42 zusammengeführt sind.

Beispielsweise und gemäß einem Aspekt der Erfindung könnten als Wirkstoff dabei einerseits ein Hydrogencarbonat, bevorzugt Kalium-, Natrium- oder Calciumhydrogencarbonat, besonders bevorzugt Natriumhydrogencarbonat, und andererseits eine Säure / Carbonsäure (z. B. Äpfelsäure, Zitronensäure, Milchsäure etc.) vorgesehen sein, vorzugsweise jeweils in wässriger Lösung und in für die angestrebte Handhabung geeigneter Konzentration. Diese werden dann gemäß dem ersten Auslegungskonzept getrennt voneinander in den behandlungsbedürftigen Raumbereich ausgebracht und gemäß dem zweiten Auslegungskonzept gemeinsam der Mischstelle 42 zugeführt, kommen jeweils dort in Kontakt miteinander und reagieren unter Bläschenbildung (CO2-Bildung). Damit kann in der Mischstelle 42 in Folge dieser Reaktion unter Bläschenbildung ein sehr hoher Druck ohne aktive Einflussnahme von außen erzeugt werden, so dass das Medium aus der Mischstelle 42 mit vergleichsweise hohem Druck und im Ergebnis als gerichteter Strahl aus der nachgeschalteten Applikationsöffnung 32 des Düsenkörpers 22 austritt. Dieser Effekt könnte beispielsweise zu Reinigungs- oder Spülzwecken besonders günstig eingesetzt werden, wobei gerade die Erzeugung eines gerichteten Strahls unter hohem Druck besonders gut nutzbar ist.

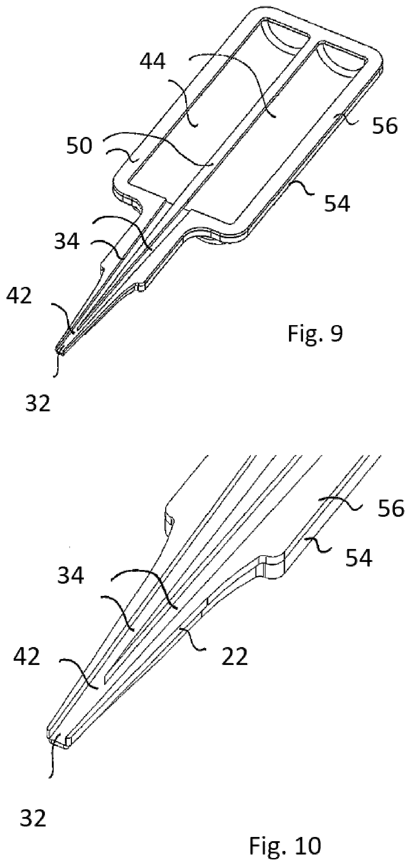

FIG. 9 und die ausschnittsweise Vergrößerung in FIG. 10 zeigen das Basalsegment des Wirkstoffapplikators 10' mit alternativer Kanalgeometrie und Kanalführung im Bereich der Mischstelle 42.

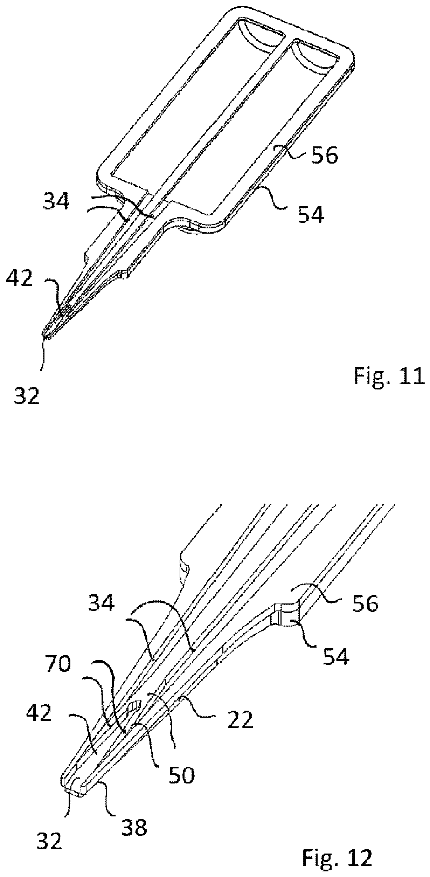

Ein weiterer als eigenständig erfinderisch angesehener Aspekt der Erfindung ist anhand der Darstellung des Basalsegments in FIG. 11 und dessen vergrößerter Darstellung in FIG. 12 gezeigt. In dieser Ausführungsform sind in die Folienschicht 56 ebenfalls zwei Medienkanäle 34 integriert, die in der Mischstelle 42 zusammengeführt sind. Hierbei sind in die Medienkanäle 34 aber kurz vor der Kontaktstelle zur Mischstelle 42 Ventilklappen 70 geschaltet. Hierbei handelt es sich im Ausführungsbeispiel um in die Folienschicht 56 eingeformte Folienstücke 50, die in der Art eines Feder- oder Blattfederventils grundsätzlich den jeweiligen Medienkanal vor der Mischstelle 42 verschließen, aber durch den Mediendruck im jeweiligen Medienkanal 34 verschoben werden können und den Kanal somit freigeben. In Gegenrichtung ist das jedoch nicht möglich, da im Falle eines erhöhten Drucks im Bereich der Mischstelle 42 die Ventilklappen 70 in ihren Ventilsitz am Rand des jeweiligen Medienkanals 34 gedrückt werden und diesen somit dichtend verschließen. Durch eine solche Ventilanordnung ist in als eigenständig erfinderisch angesehener Ausgestaltung ein Rückfluss des in der Mischstelle 42 entstehenden Mediengemisches in die Zuführ-Medienkanäle 34 ausgeschlossen, so dass gerade bei der beabsichtigten Druckerhöhung nach der Vermischung der Wirkstoffe die Ausbringung des sich bildendenden Gases nur in Richtung zum Behandlungsende 38 hin, also über die Applikationsöffnung 32, stattfindet.

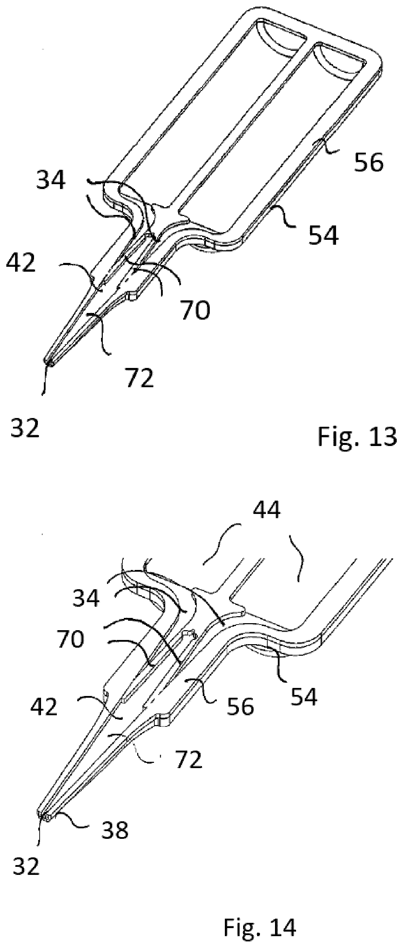

Eine weitere, ebenfalls als eigenständig erfinderisch angesehene Variante der Erfindung ist anhand der Darstellung des Basalsegments in FIG. 13 und dessen vergrößerter Darstellung in FIG. 14 gezeigt. Auch in dieser Variante sind Ventilklappen 70 entsprechend der vorstehend beschriebenen Funktionalität in die Folienschicht 56 eingearbeitet. In dieser Variante ist die Geometrie aber derart ausgestaltet, dass im Bereich der Mischstelle 42 eine Mischkammer 72 ausgeformt ist. Diese bietet insbesondere bei der vorgesehenen Vermischung von Wirkstoffen ein ausreichendes Volumen für die erwartete Gasbildung.

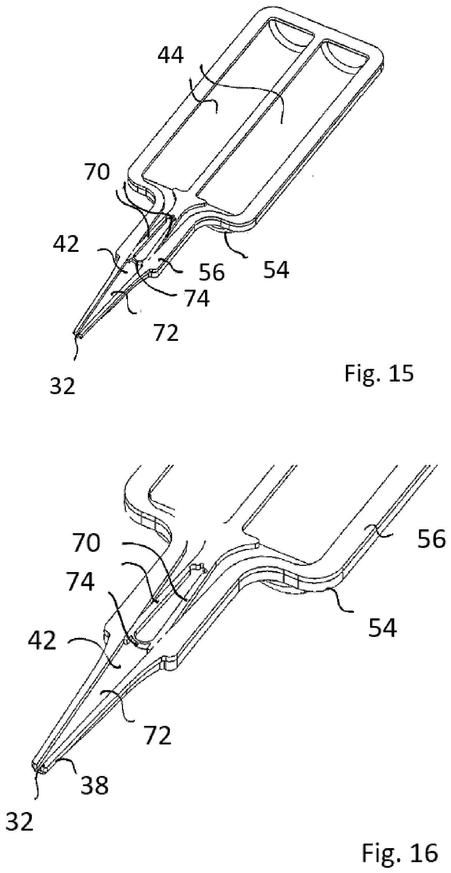

In der weiteren, in FIG. 15 (vergrößert in FIG. 16) dargestellten, ansonsten zur vorstehend beschriebenen Variante baugleichen Ausführung ist in als eigenständig erfinderisch angesehener Ausführung ein zusätzliches Federelement 74 vorgesehen, dass auf die Ventilklappen 70 einwirkt und deren Rückstell- bzw. Anpresskräfte an ihre Ventilsitze verstärkt.

Nachfolgend werden einige als erfinderisch angesehene Aspekte bezüglich der Ausgestaltung der Wirkstoffkammern 44 des Wirkstoffapplikators 10' gem. FIG. 4 näher erläutert. Dazu sind teilweise Schnitte oder Halbschnitte der perspektivischen Darstellung analog FIG. 4 gezeigt, ohne dass dabei näher auf die Kanalführung innerhalb der zentralen Folienschicht 56 eingegangen wird. Die vorstehenden Erläuterungen zur Kanalführung können aber auch bei den nachfolgenden Beispielen vollumfänglich vorgesehen sein.

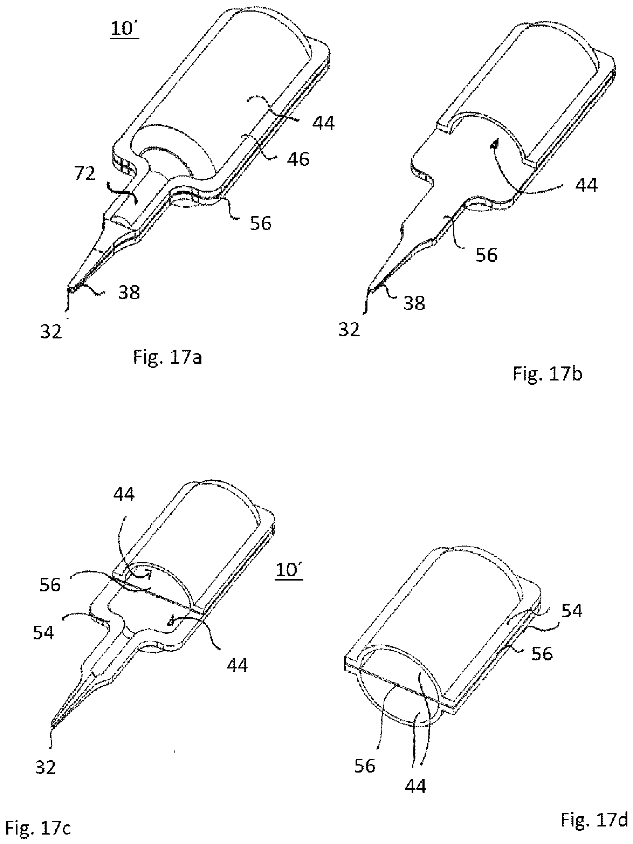

Der in FIG. 17 perspektivisch und in unterschiedlichen Teilschnitten gezeigte Wirkstoffapplikator 10' weist zwei Wirkstoffkammern 44 auf, die beidseitig der zentralen Folienschicht 56 angeordnet sind und von dieser getrennt sind. Damit können in den beiden Wirkstoffkammern 44 problemlos unterschiedliche Wirkstoffe vorgehalten werden. Des Weiteren ist in FIG. 17a deutlich erkennbar, dass im Bereich der obersten dargestellten Folienschicht 54 durch geeignete Formgebung die Mischkammer 72 ausgebildet ist. Durch die gezeigte Formgebung im Folienmaterial kann insbesondere in als eigenständig erfinderisch angesehener Ausgestaltung das Volumen der Mischkammer 72 an den vorgesehenen Zweck und den erwarteten Bedarf bei der vorgesehenen Gasbildung und dem damit einhergehenden Druckaufbau angepasst sein, im Ausführungsbeispiel ist die Mischkammer für ein Volumen von etwa 100 mm³ ausgelegt.

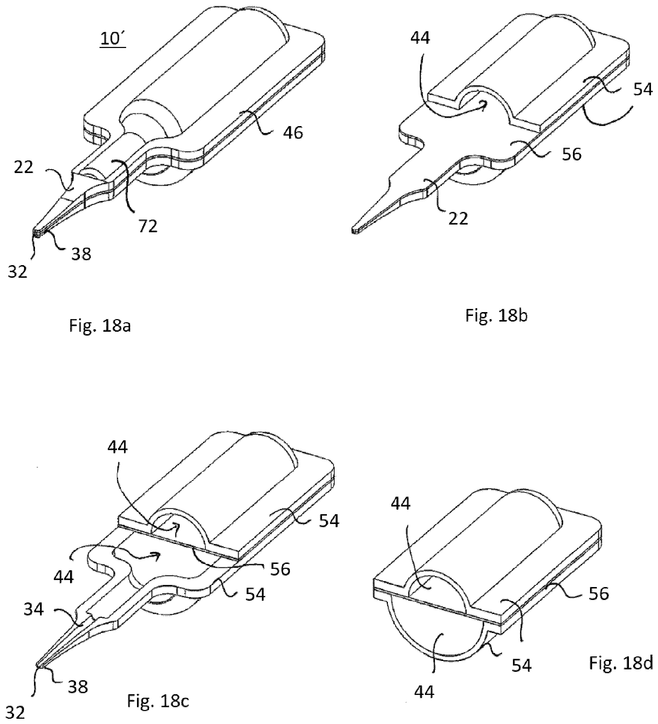

In der weiteren, In FIG. 18 gezeigten, ansonsten baugleichen Variante weist der Wirkstoffapplikator 10' Wirkstoffkammern 44 mit unterschiedlichem Innenvolumen auf. Damit können die Wirkstoffe beispielsweise in jeweils im Hinblick auf die beabsichtigte Reaktion geeignet gewählten und angepassten Mengen in den Wirkstoffkammern 44 vorgehalten werden. Auch in dieser Variante wird die Mischkammer 72 durch eine entsprechende Formgebung in der obersten Schichtfolie 54 mit einem geeignet gewählten Volumen gebildet.

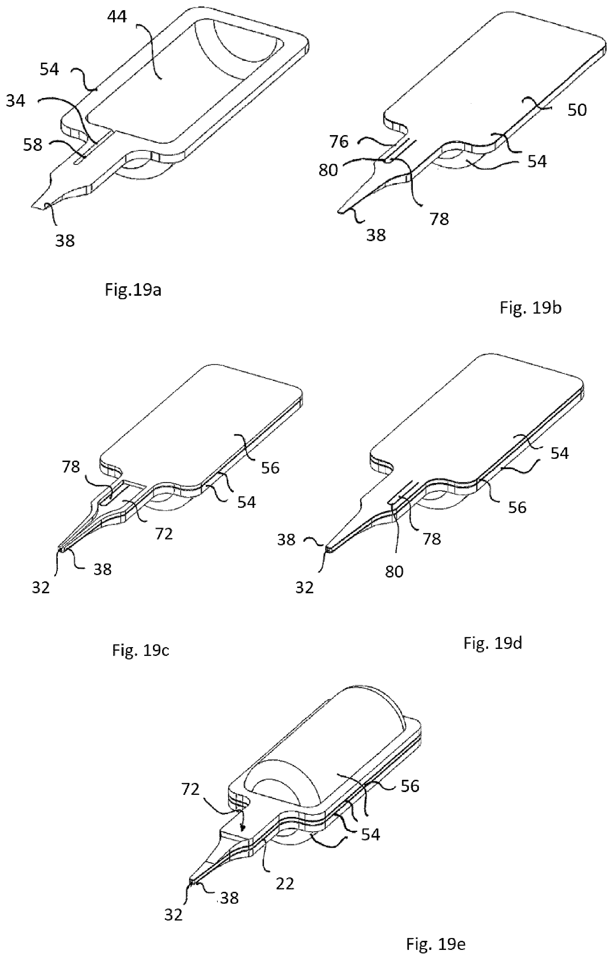

Eine weitere, als eigenständig erfinderisch angesehene Variante des Wirkstoffapplikators 10' ist in FIG. 19 anhand einer Sequenz, ausgehend von der ersten, untersten Folienschicht 54, unter schrittweiser Hinzufügung der weiteren Folienschichten 54, 56 gezeigt. Dementsprechend zeigt FIG. 19a die unterste oder erste Folienschicht 54, die in Draufsicht gesehen in ihrem Behandlungsbereich bereits an die gewünschte Form des Düsenkörpers 22 angepasst ist. Des Weiteren ist in diese Folienschicht 54 bereits, entsprechend den vorstehend beschriebenen Ausführungen, die Wirkstoffkammer 44 eingearbeitet. Das Folienstück 54 ist zudem mit einer eingeprägten Nut 58 versehen, die einen Medienkanal 34 bildet und lediglich über einen Teilbereich hinweg bis zum distalen oder Behandlungsende 38 des Applikators geführt ist.

Auf diese untere Folienschicht 54 wird anschließend beim schichtweisen Aufbau ein weiteres, ebenfalls eine Folienschicht 54 bildendes Folienstück 50 aufgebracht und beispielsweise auflaminiert. Dieser sich bildende Folienstapel ist in FIG. 19b gezeigt. Dieser umfasst somit zwei übereinanderliegende Folienschichten 54. Die zuletzt aufgebrachte Folienschicht 54 überdeckt dabei den zuvor in die untere Folienschicht 54 eingebrachten, durch die Nut 58 gebildeten Medienkanal 34 und schließt diesen grundsätzlich nach oben hin ab. In einem Übergangsbereich 76, der in seiner Position weitgehend mit dem Endbereich des in der unteren Folienschicht 54 geführten Medienkanals 34 überlappt, ist die obere Folienschicht dabei mit einer Ventilklappe 78 versehen, die beispielsweise durch Einbringung einer Durchbruchlinie 80, z. B. durch Lasern oder Stanzen, ausgebildet werden kann. Die Ventilklappe 78 ermöglicht dabei den Übertritt von im Medienkanal 34 in der unteren Folienschicht 54 geführtem Medium nach oben hin, also in die nächstfolgende obere Folienschicht 56.

Diese ist auf das entstehende Schichtpaket, wie in FIG. 19c gezeigt, aufgebracht und als zentrale Folienschicht 56 gemäß den oben erläuterten Kriterien ausgeführt. Die Folienschicht 56 kann somit die Funktion einer Stütz- oder formgebenden Schicht übernehmen, die dem gesamten Paket eine gewisse Steifigkeit und mechanische Stabilität gibt.

Deutlich ist in FIG. 19c erkennbar, dass die zentrale Folienschicht 56 in einem Bereich oberhalb der zuvor genannten Ventilklappe 78 mit einer Aussparung versehen ist, die die Mischkammer 72 ausbildet. Anschließend werden zur Vervollständigung des Wirkstoffapplikators 10', analog und symmetrisch zu den in FIG. 19a und 19b gezeigten Folienschichten 54, zwei weitere Folienschichten 54 aufgebracht, wie dies FIGs. 19d und 19e gezeigt ist. Damit ist im gezeigten Ausführungsbeispiel der Wirkstoffapplikator 10' als fünfschichtiges Folienlaminat ausgeführt und für eine Vermischung der in den beidseitig der zentralen Folienschicht 56 angeordneten Wirkstoffkammern 44 in der Mischkammer 72 in der zentralen Folienschicht 56 geeignet ausgeführt.

Alternativ und in als eigenständig erfinderisch angesehener Weise kann der Düsenkörper 22, in ansonsten zur vorstehend beschriebenen Auslegung weitgehend identischer Ausgestaltung, anstelle einer Applikationsdüse 16 auch als Zwischenstück 90 ausgestaltet sein, bei dem die Einbringung der Medien in den Mundraum des Patienten nicht über am Behandlungsende des Düsenkörpers 22 angeordnete Applikationsöffnungen 32, sondern über ein eigenständiges Ausbringungsteil 92, beispielsweise ein Schlauchpaket, einen Behandlungsschnorchel oder dergleichen, erfolgt. Bei einer solchen Ausgestaltung kann das eigentliche Ausbringungsteil 92 geeignet an den Auslassbereich des Düsenkörpers 22 angeschlossen werden, wobei ein in der Mischstelle 42 oder insbesondere in der Mischkammer 72 erzeugtes, unter Druck stehendes Behandlungsmedium über eine entsprechende, im Ausbringungsteil 92 vorgesehene Druckleitung 94 an den vorgesehenen Ausbringungsort im Mundraum des Patienten geleitet werden kann. Entsprechend können dabei für weitere im Düsenkörper 22 geführte Medien, wie beispielsweise Einzelmedien, die erst an der Ausbringungsstelle im Mundraum des Patienten in Kontakt zueinander gebracht werden sollen, geeignete weitere Medienkanäle 34 im Ausbringungsteil 92 vorgesehen sein.

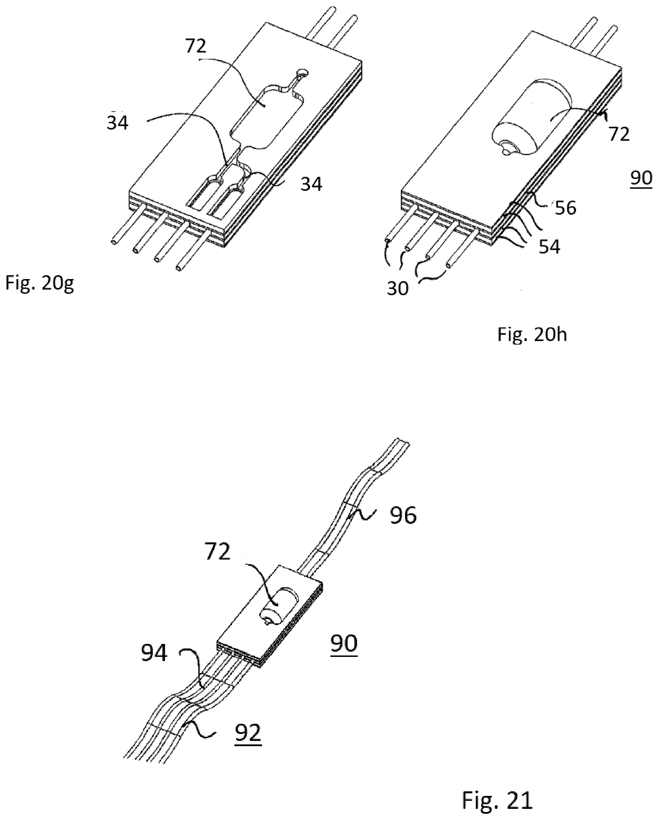

Wie der Darstellung in FIG. 20g, FIG. 20h gut entnehmbar ist, wird auch in dieser Ausführung die Mischkammer 72 in einer der oberen Folienschichten 54 angelegt und in der äußersten Folienschicht 54 durch geeignete Formgebung ausgebildet und in ihrem Volumen definiert.

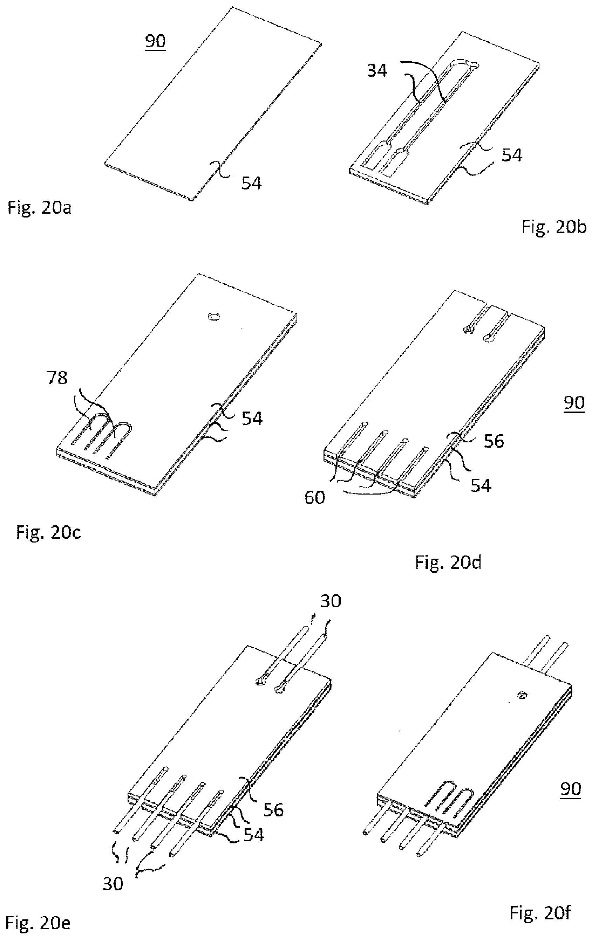

Ein solches als eigenständig erfinderisch angesehenes Zwischenstück 90 ist in FIG. 20 anhand einer Sequenz von Herstellungsschritten gezeigt. Dabei können beliebige der vorstehend beschriebenen Komponenten und Elemente zur Ausgestaltung des Zwischenstücks 90 analog vorgesehen sein. Das ein- und ausgangsseitig mit entsprechenden Medienleitungen verbundene Zwischenstück 90 ist hingegen in FIG. 21 gezeigt.

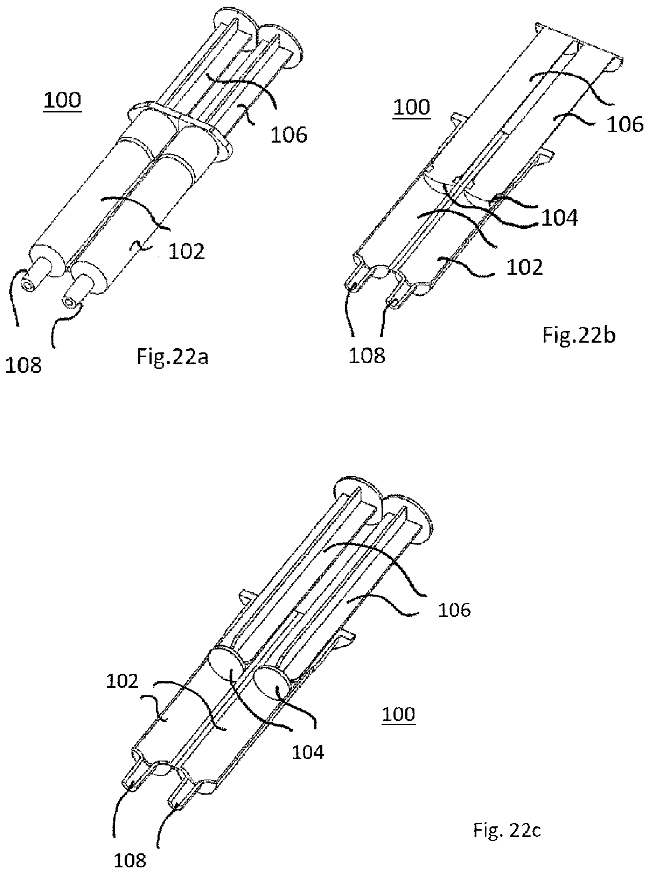

Eingangsseitig kann das Zwischenstück 90 dabei über die Medienzuleitung 96 mit geeigneten Containern für die jeweils vorgesehenen Wirkstoffe verbunden sein, wobei beispielsweise herkömmliche Karpulen oder auch Wirkstoffbehälter der vorstehend beschriebenen Art vorgesehen sein könnten. In als eigenständig erfinderisch angesehener Weise kann dabei zur Einbringung des Wirkstoffs aber auch ein Doppelspritzensystem 100 vorgesehen sein, wie es in FIG. 22 in perspektivischer Ansicht (FIG. 22a), im Längsschnitt (FIG. 22b) und im Teilschnitt (FIG. 22c) gezeigt ist. Das Doppelspritzensystem 100 umfasst dabei in an sich üblicher Ausgestaltung zwei funktional parallel geschaltete Spritzenkörper 102, die jeweils eine Wirkstoffkammer ausbilden, und in denen jeweils ein Spritzenkolben 104 geführt ist. Die Spitzenkolben 104 sind dabei jeweils von einem Betätigungsstößel 106 angetrieben, wobei die Betätigungsstößel 106 im Ausführungsbeispiel miteinander gekoppelt ausgeführt sind. Bei einer Betätigung, die manuell oder auch automatisiert in einem entsprechenden Versorgungssystem ausgeführt werden kann, werden dabei somit die in den Spritzenkörpern 102 vorgehaltenen Wirkstoffe zeitgleich über die jeweiligen Anschlussstutzen 108 ausgetragen und über die angeschlossene Medienzuleitung 96 dem Zwischenstück 90 zugeführt.

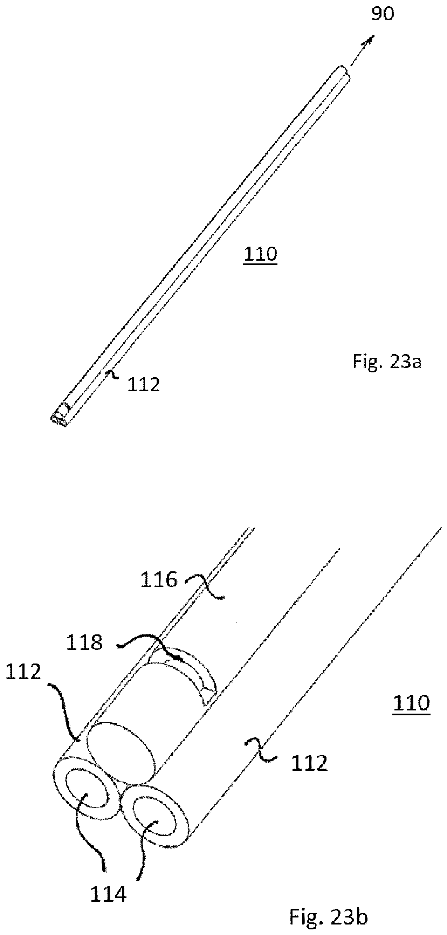

Ausgangsseitig ist das Zwischenstück 90 wie erwähnt an ein Ausbringungsteil 92 angeschlossen. Dieses kann, wie beispielhaft in FIG. 23 gezeigt, als Schlauchpaket 110 ausgeführt sein. Das Schlauchpaket 110 umfasst dabei eine Anzahl von, im Ausführungsbeispiel zwei, Wirkstoffschläuche 112, die endseitig in jeweils einer Ausbringungsöffnung 114 münden. Über diese kann der jeweilige Wirkstoff somit direkt und zielgenau an den gewünschten Ausbringungsort geliefert werden. Des Weiteren umfasst das Schlauchpaket 110 auch noch einen Druckschlauch 116, der eingansseitig an die Mischkammer 72 des Zwischenstücks 90 angeschlossen ist. Sobald somit in der Mischkammer 72 gemäß einem Aspekt der Erfindung durch gezielte Vermischung zweier Ausgangsstoffe als Reaktionsprodukt ein unter erhöhtem Druck stehendes Gas erzeugt wird, kann dieses über den Druckschlauch 116 abgeleitet und über dessen Austrittsöffnung 118 ausgebracht werden.

Wie der vergrößerten Darstellung in FIG. 23b deutlich entnehmbar ist, ist die Austrittsöffnung 118 für das erzeugte Druckgas dabei für eine seitliche Abströmrichtung ausgelegt, so dass beispielsweise bei einer zahnärztlichen Behandlung gezielt in seitlicher Richtung gereinigt werden kann.

Gemäß einem Aspekt der Erfindung kann das Zwischenstück 90 somit eingangsseitig an eine Doppelspritze 100 der gezeigten Art, an eine Doppelkarpulenspritze oder an beliebig andere geeignete Wirkstoffkammern ankoppelbar sein. Es kann je nach Bedarf und auslegungsgemäßer Verwendung Ventile, insbesondere in der Art der beschriebenen Ventilklappen 70, 78, eine Misch- oder Reaktionskammer 72 der vorstehend beschriebenen Art oder eine Kombination davon aufweisen, es ist insofern dazu geeignet, bedarfsweise einen Spülausgang und/oder einen Druckausgang aufzuweisen. Eingangsseitig kann es abhängig von der individuellen Auslegung eine geeignete Anzahl von Medieneingängen, insbesondere zwei oder vier Eingänge, aufweisen. Das Zwischenstück 90 kann sich insbesondere zwischen den Ampullen / dem Ampullensystem und dem Applizierungsteil befinden. Es umfasst beispielsweise eine reine Zusammenführung zweier Medienkanäle (z.B.: wässrige Lösung aus Carbonsäure und wässrige Lösung eines Hydrogencarbonates). Es fungiert in dieser Funktion als einfaches Y-Stück und beinhaltet mindestens einen Ausgang. Die gewünschte Wirkfunktion ist dabei eine nicht gerichtete Druckentfaltung zum Spülen.

Alternativ kann das Zwischenstück 90 einer Zusammenführung von Medien, direkt oder indirekt mündend in eine Misch- oder Reaktionskammer 72, dienen, wobei es mindestens einen Medienausgang umfasst. Die gewünschte Wirkfunktion ist in diesem Fall eine gerichtete Druckentfaltung vorzugsweise zum Reinigen einer kontaminierten Oberfläche und zum Spülen.

Beide vorangegangenen Zwischenstückfunktionen können mit und ohne Rückschlagventile ausgestaltet sein. In einer besonders günstigen Ausgestaltung des Zwischenstücks sind die genannten Wirkfunktionen und Ausgestaltungen kombiniert vorgesehen.

Die Versorgung des Zwischenstücks 90 kann auch bei einer Ausgestaltung nach vorgenannter Auslegung mit mindestens zwei Ampullen gemeinsam erfolgen, welche in dem Zwischenstück 90 medienseitig zusammengeführt werden. In einer weiteren Ausführungsvariante können die Wirkfunktionen aber auch jeweils einzeln von mindestens zwei Ampullen bereitgestellt werden.

Das Zwischenstück 90 kann auch mit dem Ampullenteil und oder dem Applizierteil einstückig sein.

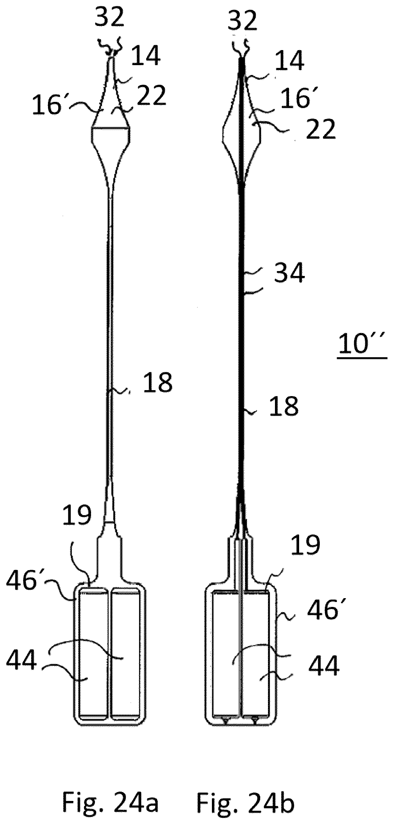

Eine alternative Ausgestaltung eines Wirkstoffapplikators 10" ist in FIG. 24 sowohl in Draufsicht (FIG. 24a) als auch im Längsschnitt (FIG. 24b) gezeigt. Dieser weist analog zur Ausführungsform gem. FIG. 4 einen Grundkörper 46' auf, in den die beiden Wirkstoffkammern 44 integriert sind. Diese sind medienseitig über ein Schlauchstück 18, das im Hinblick auf die vorgesehenen Behandlungsmethoden vergleichsweise lang ausgeführt sein kann, mit der am distalen Ende 14 angeordneten, durch den Düsenkörper 22 gebildeten Applikationsdüse 16' verbunden. Die zur medienseitigen Verbindung der Wirkstoffkammern 44 mit den Applikationsöffnungen 32 an der Applikationsdüse 16' vorgesehenen mindestens zwei Medienkanäle 34 sind dabei wie vorstehend beschrieben im Düsenkörper 22 integriert angeordnet und von diesem ausgehend innerhalb des Schlauchstücks 18 bis hin zu den Wirkstoffkammern 44 weitergeführt. Der hier gezeigte Wirkstoffapplikator 10" kann analog zum vorgenannten Beispiel einstückig ausgeführt sein und als solcher zum unmittelbaren Einsatz oder auch als Befüllung für den Wirkstoffapplikator 10 gem. FIG. 2 vorgesehen sein. Alternativ kann der Wirkstoffapplikator 10" auch mehrstückig ausgeführt sein, wobei beispielsweise das Schlauchstück 18 über geeignete Anschlüsse mit einer separat ausgeführten Applikationsdüse 16' und/oder mit einem separat ausgeführten Versorgungscontainer 19 verbunden werden könnte.

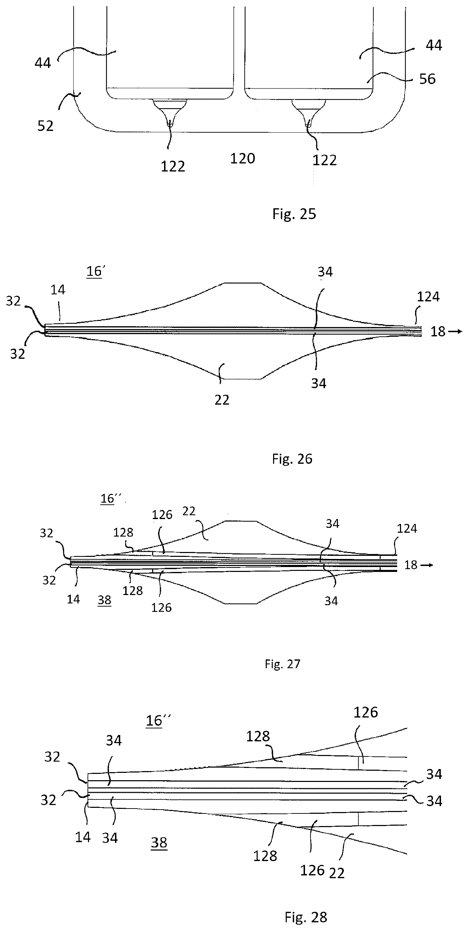

Die Wirkstoffkammern 44 in den vorstehend erläuterten Beispielen können gemäß einem Aspekt der Erfindung durch Ausformungen in den jeweiligen Folienschichten 52, 54, 56 gebildet sein. Die Befüllung mit Wirkstoff kann dann beispielsweise durch Einspritzen des Wirkstoffs zwischen die vorlaminierten Folienschichten 52, 54, 56 erfolgen, die sich dabei ggf. noch weiter verformen können und somit die eigentliche Wirkstoffkammer 44 ausbilden. Anschließend, also nach vollständiger Befüllung mit Wirkstoff, können die Folienschichten 52, 54, 56 geeignet verschweißt und/oder versiegelt werden. Der Bodenbereich der jeweiligen Wirkstoffkammer 44 mit der jeweils entsprechend verschweißten oder versiegelten Befüllungsöffnung 122 ist im Längsschnitt in FIG. 25 gezeigt.

Die Applikationsdüse 16' des Wirkstoffapplikators 10" gem. FIG. 24 ist in FIG. 26 im vergrößerten Längsschnitt gezeigt. In einem Anschlussbereich 124 für das Schlauchpaket 18 verbreitert sie sich im Querschnitt zunächst in Richtung auf das distale Ende 14 hin, bis sie in den eigentlichen Düsenkörper 22 übergeht und sich dort gemäß einem Aspekt der vorliegenden Erfindung zum distalen Ende 14 hin wieder verjüngt. Die integrierten Medienkanäle 34 sind dabei vollständig durch das gezeigte Segment hindurchgeführt.

In FIG. 27 ist eine Variante der Applikationsdüse 16''im Längsschnitt gezeigt, die zusätzlich zu den beiden Medienkanälen 34 als weitere Medienkanäle noch eine Anzahl von Absaugkanälen 126 umfasst. Diese sind analog der Medienkanäle 34 sowohl durch den Düsenkörper 22 als auch durch das daran angeschlossene Schlauchstück 18 geführt und münden im Bereich des Behandlungsendes 38 in seitlich angeordneten Absaugöffnungen 128. Die Absaugkanäle 126 sind dabei gemäß einem Aspekt der Erfindung, wie der Darstellung in FIG. 27 und auch der vergrößerten Darstellung in FIG. 28 deutlich entnehmbar ist, mit größerem Innendurchmesser ausgeführt als die Medienkanäle 34; ebenso sind die Absaugöffnungen 126 größer ausgeführt als die Applikationsöffnungen 32. Damit wird dem Umstand Rechnung getragen, dass bei der erwarteten Behandlungsmethode selbst die Einbringung einer vergleichsweise gering bemessenen Wirkstoffmenge aufgrund der angestrebten Reaktion der Wirkstoffe miteinander am Einsatzort ein vergleichsweise großer Absaugbedarf für die entstehenden Reaktionsprodukte, aber auch für infolge der Spülung oder Reinigung angefallene Fragmente, Speisereste und sonstige Reinigungsprodukte entstehen kann und sollte. Die Absaugung kann aber auch in einer hier nicht dargestellten Variante örtlich gesehen weiter weg von den Applikationsöffnungen 32 oder dem Düsenkörper 22 angeordnet sein. Vorteilhafter Weise ist/sind die Absaugung/en als eine Art Glocke aus einem weichen material gestaltet. Hierbei besteht die Möglichkeit, dass diese Glocke nach dem Einbringen der Applikationsdüse 16 in die Zahntasche 8 diese von außen verschließt, um in einer besonders effizienten Weise die aus der Zahntasche 8 ausgeschwämmten Bestandteile absaugen zu können.

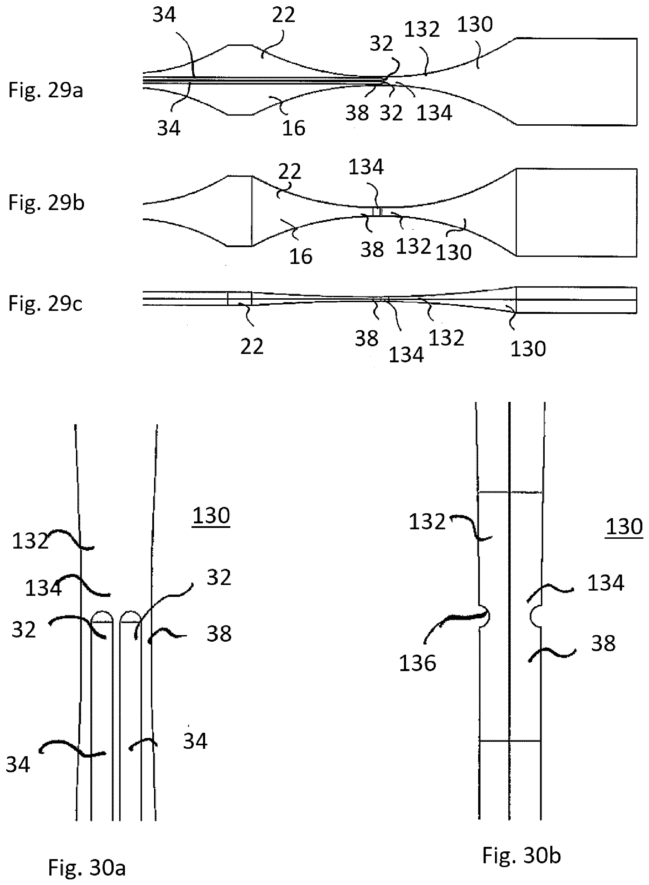

Gemäß einem weiteren, als eigenständig erfinderisch angesehene Aspekt sind die Wirkstoffkammern 44 zum zugeordneten Medienkanal 32 hin oder auch der jeweilige Medienkanal 34 nach außen hin durch ein Siegel- oder Abschlusselement 130 verschlossen, so dass ein unbeabsichtigtes Austreten des Wirkstoffs vermieden ist. In FIG. 29 ist ein solches Abschlusselement 130 für das Beispiel des Wirkstoffapplikators 10" gem. FIG. 24 im Längsschnitt (FIG. 29a), in Draufsicht (FIG. 29b) und in seitlicher Ansicht (FIG. 29c) gezeigt; selbstverständlich ist diese Ausführungsform aber auch für die anderen Varianten der Wirkstoffapplikatoren 10, 10' geeignet. Dabei ist an das Behandlungsende 38 des Düsenkörpers 22 ein abreissbares Verschlusselement 132 angeformt, das die austrittseitigen Enden der Medienkanäle 34 und ggf. der Absaugkanäle 126, also insbesondere die Applikationsöffnungen 32 bzw. die Absaugöffnungen 128 geeignet verschließt. Wie insbesondere der vergrößerten Darstellung in FIG. 30 entnehmbar ist, ist das Verschlusselement 132 dabei an den Auslassbereich des Düsenkörpers 22 über eine Sollbruchstelle 134, im Ausführungsbeispiel eine geeignete Schwächung im Grundmaterial, angeformt. An dieser Sollbruchstelle 134 kann das Verschlusselement 132 abgerissen werden, wobei die Applikationsöffnungen 32 und ggf. die Absaugöffnungen 128 freigelegt und die Medienkanäle 34 bzw. Absaugkanäle 126 zugänglich gemacht werden.

Insbesondere ist zu erkennen, dass im Zustand mit angebrachtem Verschlusselement, wie in FIG. 30a dargestellt, die Medienkanäle 34 verschlossen sind.

FIG. 30b zeigt die Seitenansicht der Sollbuchstelle 134, auf welcher zu erkennen ist, dass im Bereich der Sollbruchstelle 134 die Materialstärke in Gestalt eine Art Kerbe 136 stark reduziert ist. Die verschlossenen Medienkanäle 34 reichen dabei über die Sollbruchstelle 134 hinaus. Wird nun das Verschlusselement 132 von dem Wirkstoffapplikator 10‴ nach FIG 24 durch Ziehen oder Reißen separiert, bilden sich an den Medienkanälen 34 der Applikationsdüse 16 zwei Applikationsöffnungen 32. Der Vorteil besteht insbesondere darin, dass die im Vorfeld sterile Applikationsdüse 16‴ nach dem Öffnen der Medienkanäle 34 weiterhin steril bleiben kann.

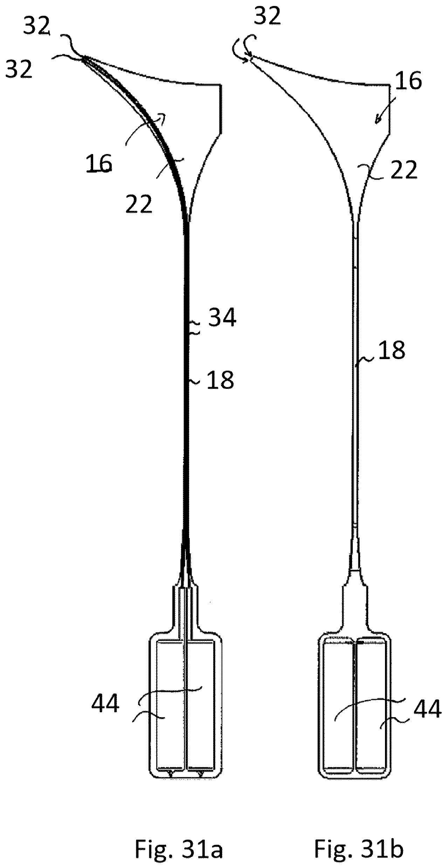

Die bisherigen Beispiele betreffen Wirkstoffapplikatoren mit im Wesentlichen gerade ausgerichteter Applikationsdüse 16. Diese kann alternativ aber auch seitlich, abgeschrägt oder gekrümmt ausgeführt sein, um ggf. die Handhabung zu erleichtern. Dies ist beispielhaft für den Wirkstoffapplikator 10" in FIG. 31 gezeigt.

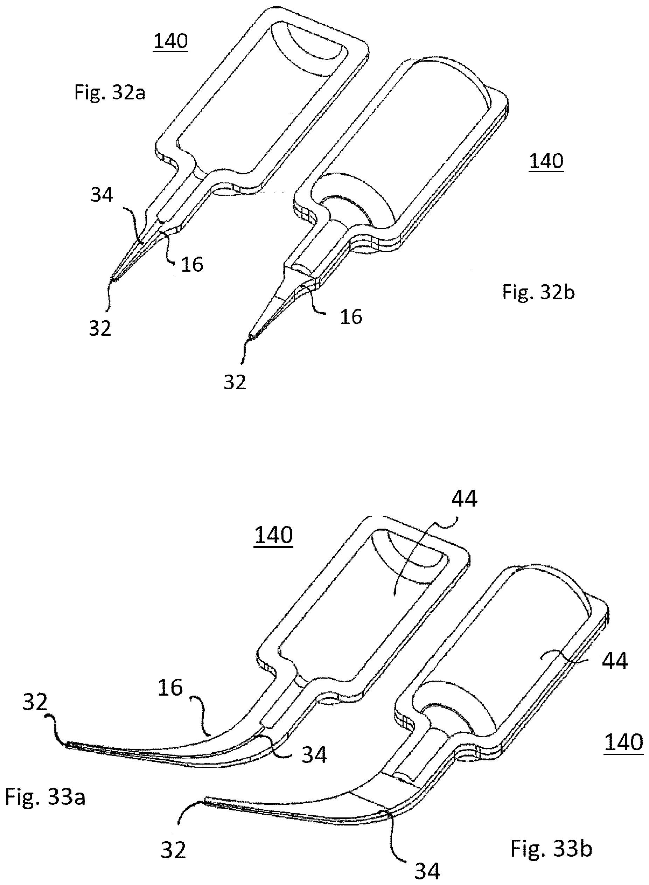

Gemäß einem als eigenständig erfinderisch angesehenen Aspekt kann grundsätzlich die Bereitstellung eines Hydrogencarbonats als Wirkstoff in einer über einen Medienkanal 34 mit einer Applikationsöffnung 32 verbundenen Wirkstoffkammer 44 auch im Rahmen lediglich einer Wirkstoffkammer 44 vorgesehen sein. Ein solchermaßen aufgebauter Wirkstoffapplikator 140, in dessen Wirkstoffkammer 44 ein ein Hydrogencarbonat vorgehalten ist, ist mit geradlinig ausgerichteter Applikationsdüse in FIG. 32 in Teilansicht (FIG. 32a) und in perspektivischer Ansicht (FIG. 32b) und mit in seitlicher Richtung gekrümmt ausgerichteter Applikationsdüse in FIG. 33 in Teilansicht (FIG. 33a) und in perspektivischer Ansicht (FIG. 33b) gezeigt.

### Bezugszeichenliste

- 1: Zahn
- 2: Kieferknochen
- 4: Zahnhals
- 6: Zahntasche
- 8: Weichgewebe
- 10, 10', 10": Wirkstoffapplikator
- 11: Schlauchventil
- 12: Griffstück
- 14: Ende
- 15: Fixierungsstück
- 16, 16': Applikationsdüse
- 17: Schutzhülle
- 18: Schlauchstück
- 19: Versorgungscontainer
- 20: Steuereinheit
- 21: Stromversorgungseinheit
- 22: Düsenkörper
- 23: mechanisches, pneumatisches oder hydraulisches Drucksystem
- 30: Zugangsstutzen
- 32: Applikationsöffnung
- 34: Medienkanal
- 36: Anschlussseite
- 38: Behandlungsende
- 40: Ausströmbereich
- 42: Mischstelle
- 44: Wirkstoffkammer
- 46, 46': Grundkörper
- 48: Verschlusselement
- 50: Folienstück
- 52, 54, 56: Folienschicht
- 58: Nut
- 60: Austiefungen
- 62: Aussparung
- 64: Übertrittspunkt
- 70: Ventilklappen
- 72: Mischkammer
- 74: Federelement
- 76: Übergangsbereich
- 78: Ventilklappe
- 80: Durchbruchlinie
- 90: Zwischenstück
- 92: Ausbringungsteil
- 94: Druckleitung
- 96: Medienzuleitung
- 100: Doppelspritzensystem
- 102: Spritzenkörper
- 104: Spritzenkolben
- 106: Betätigungsstößel
- 108: Anschlussstutzen
- 110: Schlauchpaket
- 112: Wirkstoffschlauch
- 114: Ausbringungsöffnung
- 116: Druckschlauch
- 118: Austrittsöffnung
- 120: Bodenbereich
- 122: Befüllungsöffnung
- 124: Anschlussbereich
- 126: Absaugkanal
- 128: Absaugöffnung
- 130: Abschlusselement
- 132: Verschlusselement
- 134: Sollbruchstelle
- 136: Kerbe
- 140: Wirkstoffapplikator
- d: Foliendicke
- D: Gesamtdicke

## Patentansprüche

1. Applikationsdüse (16, 16') zur Ausbringung eines dentalen Wirkstoffs im Mundraum eines Patienten, mit einem sich in einer Längsrichtung von einem Anschlussbereich (36) aus zu einem freien Behandlungsende (38) hin erstreckenden Düsenkörper (22), in den mindestens zwei unabhängig voneinander vom Anschlussbereich (36) ausgehende zum freien Behandlungsende hin verlaufende Medienkanäle (34) integriert sind, **dadurch gekennzeichnet, dass** der Düsenkörper (22) als Laminat aus einer Mehrzahl von Folienstücken (50) aufgebauter Schichtenkörper ausgebildet ist.

2. Applikationsdüse (16, 16') nach Anspruch 1, deren Medienkanäle (34) jeweils auslassseitig in jeweils einer Applikationsöffnung (32) münden.

3. Applikationsdüse (16, 16') nach Anspruch 1 oder 2, bei dem die den beiden Medienkanälen (34) zugeordneten Applikationsöffnungen (32) mindestens 0,01 mm und höchstens 10 mm, vorzugsweise mindestens 0,1 mm und/oder höchstens 2 mm, voneinander beabstandet sind.

4. Applikationsdüse (16, 16') nach einem der Ansprüche 1 bis 3, bei der die Applikationsöffnungen (32) in einem im Bereich des Behandlungsendes (38) angeordneten Ausströmbereich (40) des Düsenkörpers angeordnet sind.

5. Applikationsdüse (16, 16') nach Anspruch 1, dessen mindestens zwei Medienkanäle (34) unabhängig voneinander vom Anschlussbereich (36) hin zu einer sie medienseitig verbindenden Mischstelle (42) verlaufen, wobei die Mischstelle (42) auslassseitig über einen weiteren Medienkanal (34) mit einer in einem im Bereich des Behandlungsendes (38) angeordneten Ausströmbereich (40) vorgesehenen Applikationsöffnung (32) verbunden ist.

6. Applikationsdüse (16, 16') nach Anspruch 5, deren Mischstelle (42) als Mischkammer (72) ausgestaltet ist, zu der hin die Medienkanäle (34) verlaufen und in die sie münden.

7. Applikationsdüse (16, 16') nach Anspruch 6, deren Mischkammer (72) ein Volumen von höchstens 1000 mm³, vorzugsweise von höchstens 500 mm³, besonders bevorzugt von höchstens 100 mm³, aufweist.

8. Applikationsdüse (16, 16') nach einem der Ansprüche 5 bis 7, mit einem zusätzlich zu den Medienkanälen (34) vorgesehenen, ebenfalls in den Düsenkörper (22) integrierten und mit der Mischstelle (42) verbundenen Druckkanal.

9. Applikationsdüse (16, 16') nach einem der Ansprüche 1 bis 8, deren Düsenkörper (22) sich flächig erstreckt und sich in seinem Querschnitt in Richtung auf das freie Behandlungsende (38) hin verjüngt.

10. Applikationsdüse (16, 16') nach einem der Ansprüche 1 bis 9, bei der die Medienkanäle (34) in einer Folienschicht (52, 54, 56) des Laminats durch eine eingebrachte Aussparung in der jeweiligen Schichtfolie (50) gebildet sind.

11. Applikationsdüse (16, 16') nach einem der Ansprüche 1 bis 10, deren Medienkanäle (34) mit integrierten Abstandshaltern versehen sind.

12. Applikationsdüse (16, 16') nach einem der Ansprüche 1 bis 11 deren Düsenkörper (22) aus mindestens drei Folienschichten (52, 54, 56) aufgebaut ist, wobei eine zentrale, zwischen zwei benachbarten Folienschichten (52, 54) angeordnete Folienschicht (56) aus einem härteren Folienmaterial gebildet ist als die beiden benachbarten Folienschichten (52, 54).

13. Applikationsdüse (16, 16') nach einem der Ansprüche 1 bis 12, deren Düsenkörper (22) aus mindestens drei Folienschichten (52, 54, 56) aufgebaut ist, in die jeweils ein Lichtleiter integriert ist.

14. Applikationsdüse (16, 16') nach einem der Ansprüche 1 bis 13, mit einem zusätzlich zu den Medienkanälen (34) vorgesehenen, ebenfalls in den Düsenkörper (22) integrierten Absaugkanal.

15. Applikationsdüse (16, 16') nach einem der Ansprüche 1 bis 14, deren Düsenkörper (22) in Draufsicht eine in der Art eines Dreiecks geformte Kontur aufweist.

16. Applikationsdüse (16, 16') nach einem der Ansprüche 1 bis 15, die als Einmal-Produkt ausgelegt ist.

17. Wirkstoffapplikator (10, 10', 10") zur Ausbringung eines dentalen Wirkstoffs im Mundraum eines Patienten, mit einer Applikationsdüse (16, 16') nach einem der Ansprüche 1 bis 16, wobei jeweils ein Medienkanal (34) medienseitig jeweils mit einer separat zugeordneten Wirkstoffkammer (44) verbunden ist.

18. Wirkstoffapplikator (10, 10', 10") nach Anspruch 17, bei dem die Wirkstoffkammern (44) in einen den Düsenkörper (22) umfassenden gemeinsamen Grundkörper (46, 46') integriert sind.

19. Wirkstoffapplikator (10, 10', 10") nach Anspruch 17 oder 18, bei dem die jeweilige Wirkstoffkammer (44) ein an die zu verabreichende Patientendosis angepasstes Innenvolumen aufweist.

20. Wirkstoffapplikator (10, 10', 10")nach einem der Ansprüche 17 bis 19, wobei jeweils ein Medienkanal (34) medienseitig jeweils mit einer separat zugeordneten Wirkstoffkammer (44) verbunden ist, bei dem eine der Wirkstoffkammern (44) mit einem Hydrogencarbonat befüllt ist.

21. Wirkstoffapplikator (10, 10', 10") nach Anspruch 20, bei dem eine der Wirkstoffkammern (44) mit einer Säure, insbesondere Äpfel-, Zitronen- oder Milchsäure, befüllt ist.

## Claims

1. An application nozzle (16, 16') for discharging a dental active substance in the oral cavity of a patient, the application nozzle having a nozzle body (22), which extends in a longitudinal direction from a connection region (36) to a free treatment end (38) and into which at least two media channels (34) running independently of one another starting from the connection region (36) to the free treatment end are integrated, **characterised in that** the nozzle body (22) is formed as a layered body in the form of a laminate built up from a plurality of film pieces (50).

2. The application nozzle (16, 16') according to claim 1, the media channels (34) of which open out in each case on the outlet side into one application opening (32) each.

3. The application nozzle (16, 16') according to claim 1 or 2, in which the application openings (32) associated with the two media channels (34) are distanced from one another at least by 0.01 mm and at most 10 mm, preferably at least 0.1 mm and/or at most 2 mm.

4. The application nozzle (16, 16') according to one of claims 1 to 3, in which the application openings (32) are arranged in an outflow region (40) of the nozzle body, which outflow region is arranged in the region of the treatment end (38).

5. The application nozzle (16, 16') according to claim 1, at least two media channels (34) of which run independently of one another from the connection region (36) to a mixing point (42) connecting them on the media side, wherein the mixing point (42) is connected on the outlet side via a further media channel (34) to an application opening (32) provided in an outflow region (40) arranged in the region of the treatment end (38).

6. The application nozzle (16, 16') according to claim 5, the mixing point (42) of which is embodied as a mixing chamber (72), towards which the media channels (34) run and into which they open out.

7. The application nozzle (16, 16') according to claim 6, the mixing chamber (72) of which has a volume of at most 1000 mm³, preferably of at most 500 mm³, particularly preferably of at most 100 mm³.

8. The application nozzle (16, 16') according to one of claims 5 to 7, with a pressure channel which is provided additionally to the media channels (34), is likewise integrated into the nozzle body (22), and is connected to the mixing point (42).

9. The application nozzle (16, 16') according to one of claims 1 to 8, the nozzle body (22) of which extends flat and tapers in its cross-section in the direction of the free treatment end (38).

10. The application nozzle (16, 16') according to one of claims 1 to 9, in which the media channels (34) are formed in a film layer (52, 54, 56) of the laminate by a cutout made in the respective layer film (50).

11. The application nozzle (16, 16') according to one of claims 1 to 10, the media channels (34) of which are provided with integrated spacers.

12. The application nozzle (16, 16') according to one of claims 1 to 11, the nozzle body (22) of which is built up from at least three film layers (52, 54, 56), wherein a central film layer (56) arranged between two adjacent film layers (52, 54) is formed from a harder film material than the two adjacent film layers (52, 54).

13. The application nozzle (16, 16') according to one of claims 1 to 12, the nozzle body (22) of which is built up from at least three film layers (52, 54, 56), into each of which there is integrated a light guide.

14. The application nozzle (16, 16') according to one of claims 1 to 13, with a suction channel provided additionally to the media channels (34) and likewise integrated into the nozzle body (22).

15. The application nozzle (16, 16') according to one of claims 1 to 14, the nozzle body (22) of which, in plan view, has a contour shaped in the manner of a triangle.

16. The application nozzle (16, 16') according to one of claims 1 to 15, which is designed as a single-use product.

17. An active substance applicator (10, 10', 10") for discharging a dental active substance in the oral cavity of a patient, the applicator having an application nozzle (16, 16') according to one of claims 1 to 16, wherein each media channel (34) is connected on the media side in each case to a separately associated active substance chamber (44).

18. The active substance applicator (10, 10', 10") according to claim 17, in which the active substance chambers (44) are integrated into a common main body (46, 46') comprising the nozzle body (22).

19. The active substance applicator (10, 10', 10") according to claim 17 or 18, in which the respective active substance chamber (44) has an inner volume adapted to the patient dose to be administered.

20. The active substance applicator (10, 10', 10") according to one of claims 17 to 19, wherein each media channel (34) is connected on the media side in each case to a separately associated active substance chamber (44), in which one of the active substance chambers (44) is filled with a hydrogen carbonate.

21. The active substance applicator (10, 10', 10") according to claim 20, in which one of the active substance chambers (44) is filled with an acid, in particular malic, citric or lactic acid.

## Revendications

1. Buse d'application (16, 16') destinée à l'application d'un principe actif dentaire dans la bouche d'un patient, avec un corps de buse (22) s'étendant longitudinalement d'une zone de raccordement (36) jusqu'à une extrémité libre de traitement (38), dans lequel sont intégrés au moins deux canaux de fluide (34) s'étendant indépendamment l'un de l'autre de la zone de raccordement (36) à l'extrémité libre de traitement, **caractérisée en ce que** le corps de buse (22) est réalisé sous forme d'un stratifié composé d'une corps stratifiés composés d'une pluralité de morceaux de film (50).

2. Buse d'application (16, 16') selon la revendication 1, dont les canaux de fluide (34) débouchent respectivement côté sortie dans une ouverture d'application (32).

3. Buse d'application (16, 16') selon la revendication 1 ou 2, dans laquelle les ouvertures d'application (32) attribuées aux deux canaux de fluide (34) sont espacées au moins 0,01 mm et au maximum 10 mm, de préférence au moins 0,1 mm et/ou au maximum 2 mm.

4. Buse d'application (16, 16') selon l'une des revendications 1 à 3, dans laquelle les ouvertures d'application (32) sont disposées dans une zone d'écoulement (40) du corps de buse pratiquée dans la zone de fin du traitement (38).

5. Buse d'application (16, 16') selon la revendication 1, dont au moins deux canaux de fluide (34) s'étendent indépendamment l'un de l'autre de la zone de raccordement (36) jusqu'à un point de mélange (42) qui les relie, du côté du fluide, le point de mélange (42) étant relié, côté sortie, par un autre canal de fluide (34), à un orifice d'application (32) pratiqué dans la zone d'écoulement (40) dans la zone de fin du traitement (38).

6. Buse d'application (16, 16') selon la revendication 5, dont le point de mélange (42) est conçu comme une chambre de mélange (72) vers laquelle les canaux de fluide (34) s'étendent et dans laquelle ils débouchent.

7. Buse d'application (16, 16') selon la revendication 6, dont la chambre de mélange (72) a un volume maximal de 1000 mm³, de préférence de 500 mm³, particulièrement de préférence de 100 mm³.

8. Buse d'application (16, 16') selon l'une des revendications 5 à 7, avec un canal de pression prévu en plus des canaux de fluide (34), également intégré dans le corps de buse (22) et relié au point de mélange (42).

9. Buse d'application (16, 16') selon l'une des revendications 1 à 8, dont le corps de buse (22) s'étend sur toute la surface et se rétrécit dans sa section transversale en direction de l'extrémité libre de traitement (38).

10. Buse d'application (16, 16') selon l'une des revendications 1 à 9, dans laquelle les canaux de fluide (34) sont formés dans une couche de film (52, 54, 56) du stratifié par un évidement pratiqué dans le film stratifié respectif (50).

11. Buse d'application (16, 16') selon l'une des revendications 1 à 10, dont les canaux de fluide (34) sont équipés d'entretoises intégrées.

12. Buse d'application (16, 16') selon l'une des revendications 1 à 11, dont le corps de buse (22) est constitué d'au moins trois couches de film (52, 54, 56), une couche centrale de film (56) disposée entre deux couches adjacentes de film (52, 54) étant constituée d'un matériau de film plus dur que les deux couches adjacentes de film (52, 54).

13. Buse d'application (16, 16') selon l'une des revendications 1 à 12, dont le corps de buse (22) est constitué d'au moins trois couches de film (52, 54, 56), dans lesquelles un guide optique est respectivement intégré.

14. Buse d'application (16, 16') selon l'une des revendications 1 à 13, avec un conduit d'aspiration prévu en plus des canaux de fluide (34), également intégré dans le corps de buse (22).

15. Buse d'application (16, 16') selon l'une des revendications 1 à 14, dont le corps de buse (22) présente un contour en forme de triangle en vue de dessus.

16. Buse d'application (16, 16') selon l'une des revendications 1 à 15, conçue comme un produit à usage unique.

17. Application de principe actif (10, 10', 10") pour l'application d'une substance active dentaire dans la bouche d'un patient, avec une buse d'application (16, 16') selon l'une des revendications 1 à 16, un canal de fluide (34) étant respectivement relié, du côté du fluide, à une chambre de principe actif (44) associée séparément.

18. Application de principe actif (10, 10', 10") selon la revendication 17, dans laquelle les chambres de principe actif (44) sont intégrées dans un corps de base commun (46, 46') englobant le corps de buse (22).

19. Application de principe actif (10, 10', 10") selon la revendication 17 ou 18, dans laquelle la chambre de principe actif respective (44) présente un volume interne adapté à la dose à administrer au patient.

20. Applicateur de substance active (10, 10', 10") selon l'une des revendications 17 à 19, dans lequel un canal de fluide (34) est respectivement relié, du côté du fluide, à une chambre de principe actif associée séparément (44), dans lequel l'une des chambres de principe actif (44) est remplie d'un carbonate d'hydrogène.

21. Applicateur de substance active (10, 10', 10") selon la revendication 20, dans lequel l'une des chambres de principe actif (44) est remplie d'acide, notamment d'acide malique, citrique ou lactique.
